# EUROPEAN PATENT SPECIFICATION

(11) **EP 2 041 580 B1**
(45) Date of publication and mention of the grant of the patent: **09.02.2011**
(21) Application number: 07786032.8
(22) Date of filing: 12.07.2007
(51) Int. Cl.: G01N 33/94, G01N 33/543

(54) **HOMOGENEOUS DOUBLE RECEPTOR AGGLUTINATION ASSAY FOR IMMUNOSUPPRESSANT DRUGS**
HOMOGENER DOPPELREZEPTOR-AGGLUTINATIONSTEST FÜR IMMUNSUPPRESSIVA
TEST D'AGGLUTINATION HOMOGÈNE SUR DOUBLE RÉCEPTEUR POUR MÉDICAMENTS IMMUNOSUPPRESSEURS

(30) Priority: 13.07.2006 US 807245 P; 03.07.2007 US 772886
(43) Date of publication of application: 01.04.2009
(73) Proprietor: Roche Diagnostics GmbH, 68305 Mannheim (DE); F. Hoffmann-La Roche AG, 4070 Basel (CH)
(72) Inventor: SIGLER, Gerald, F., Carmel, IN 46033 (US); GHOSHAL, Mitali, Fishers,IN 46038 (US); RASHID, Shaker, Fishers, IN 46038 (US); WU, Yifei, Westfoeld, IN 46070 (US); DORN, Allan, R., Carmel, IN 46033 (US); TSAI, Jane, S., C., Indianapolis, IN 46256 (US)
(74) Representative: Schwarz, Ralf
(86) International application number: PCT/EP2007/006200
(87) International publication number: WO 2008/006588

(56) References cited:
- EP-A- 1 239 285
- WO-A-2005/088307
- WO-A-2006/108130
- WO-A-2007/028580
- US-B1- 6 187 547
- US-B1- 6 338 946
- ARMSTRONG VICTOR W ET AL: "Modified pentamer formation assay for measurement of tacrolimus and its active metabolites: Comparison with liquid chromatography-tandem mass spectrometry and microparticle enzyme-linked immunoassay (MEIA-II)" CLINICAL CHEMISTRY, vol. 44, no. 12, December 1998 (1998-12), pages 2516-2523, XP002453941 ISSN: 0009-9147 cited in the application

## Description

### Field of the invention

The present invention pertains to the field of therapeutic drug monitoring, and in particular, to immunoassay methods for determining the presence or amount of immunosuppressive drug substances in a sample. More particularly, the present invention relates to homogeneous, non-competitive, double receptor assays for measuring immunosuppressant drugs.

### Background

Several important immunosuppressive drugs act on immunophilins, cyclosporin, tacrolimus, rapamycin, and everolimus. Immunophilins are protein chaperones with peptidylprolyl isomerase activity that belong to one of the two large families, the cyclosporin-binding cyclophilins (CyPs) and the FK506 binding proteins (FKBPs).

Cyclosporin (also known as ciclosporine and cyclosporin A) is natural metabolite of a soil fungus, a peptide composed of 11 amino acids and, together with tacrolimus, is a calcineurin inhibitor. Cyclosporin is thought to bind to the cytosolic protein cyclophilin (an immunophilin) of immunocompetent lymphocytes, especially T-lymphocytes. This complex of cyclosporin and cyclophilin inhibits calcineurin, which under normal circumstances induces the transcription of interleukin-2. The drug also inhibits lymphokine production and interleukin release, leading to a reduced function of effector T-cells.

Tacrolimus (FK506) is also a fungal product *(Streptomyces tsukubaensis).* It is a macrolide lactone and acts by inhibiting calcineurin. The drug is used particularly in liver, kidney, heart, and lung transplants. It binds to an immunophilin, followed by the binding of the complex to calcineurin and the inhibition of its phosphatase activity. In this way, it prevents the passage of G0 into G1 phase. Tacrolimus is more potent than cyclosporine and has less pronounced side effects.

Rapamycin (sirolimus) is an extremely potent immunosuppressive drug. It forms a tight complex with FKBP12 (a 12-kDa FK506 binding protein), and the rapamycin-FKBP12 complex in turn binds to mammalian target of rapamycin (mTOR) or yeast target of rapamycin (yTOR). There are two forms of yTOR, yTOR1 and yTOR2. As a member of the novel phosphatidylinositol kinase-related kinase family, mTORs kinase activity is essential for its signaling function. The FKBP12-rapamycin binding domain (FRBD) in mTOR has been identified as an 11 kDa segment located N-terminal to the kinase domain. Although cyclosporin and tacrolimus (FK506) have different molecular structures, their immunosuppressive properties and molecular mechanisms are similar. Both drugs bind intracellularly to abundant cytosolic proteins known as immunophilins. In the case of tacrolimus, the major binding protein appears to be FKBP12, while cyclosporin binds to a family of immunophilins called cyclophilins. These drug-immunophilin complexes can then form a pentameric complex with calcineurin, calmodulin, and calcium causing a non-competitive inhibition of calcineurin-phosphatase activity.

Everolimus is an immunosuppressive macrolide bearing a stable 2-hydroxyethyl chain at position C-40 on the sirolimus structure. Everolimus, which has greater polarity than sirolimus, was developed in an attempt to improve the pharmacokinetic characteristics of sirolimus.

Calcineurin (CaN), a heterodimeric protein phosphatase, is composed of a 61 kDa catalytic subunit A (CaNA) and a 19 kDa regulatory subunit B (CaNB). It is known that the inhibitory effect of tacrolimus (FK506) on CaN enzyme activity is largely mediated through FKBP12. The fact that the FK506/FKBP12 complex binds CaN with high affinity in the presence of calcium makes it possible to develop a two-receptor "sandwich" assay to quantitatively measure tacrolimus in blood samples.

Since crystal structural analysis has revealed that both CaN A and B contribute interaction interfaces for binding to the FK506-FKBP12 complex, the desired fusion ought to span these two subunits. Recently, expression and purification of a 97 kDa single-chain fusion calcineurin A/B-calmodulin (scCN) polypeptide has been reported (Y. Qin et al, Biochimica et Biophysica Acta, 1747, 171-178, 2005). Although it was implicated that the single-chain CaN-CaM may exhibit phosphatase activity, the ability to bind FK506-FKBP12 complex has not yet been determined. In addition, the expression level of this 97 kDa scCN was quite poor. In an effort to produce a smaller and easier to manipulate protein, Schreiber and co-workers reported the generation of truncated CaN fusions. In one of such fusions, the amino acids 12-394 of CaNA were fused to the N-terminal of entire CaNB in order to express a functional CAB (fCAB). Similarly, a shorter version called minimal CAB (mCAB) and encompassing the amino acids 340-394 of CaNA and the entire CaNB, was also constructed. The binding activity of these fCAB and SCAB to FK506-FKBP12 complex was indicated by the transcriptional reporter assays in the transiently transfected Jurkat T cells (P. Clemons et al, Chemistry & Biology, 9, 49-61, 2002). However, no further studies have been reported since then regarding the expression and purification of the fCAB and mCAB to directly determine the interaction with FK506-FKBP12 in vitro.

For the adequate management of transplant patients on immunosuppressant drug therapy, it is important to obtain optimal blood concentration of the drug. Since immunosuppressant drugs are often given in combination with each other, specificity for the parent drug is critical.

Competitive immunoassays for immunosuppressant drugs in which a drug analog conjugate competes with free drug for a limited number of antibody binding sites are known and commercially available. Although these immunoassays show good selectivity for parent drug, they generally show significant cross-reactivity to one or more metabolites, thus giving rise to undesirable positive bias in the immunoassays when compared to reference methods. Current assays for immunosuppressant drugs also include high performance liquid chromatography coupled to mass spectrometry (LC/MS/MS). These methodologies require special equipment and skills.

A further problem is seen with competitive immunoassays of, e.g., rapamycin, which require the use of an analog derivative of rapamycin, which is inherently unstable. A further problem with competitive immunoassays is that it is difficult to achieve sufficient sensitivity to measure very low therapeutic ranges, e.g., 5-20 ng/ml.

Direct covalent coupling of binding protein to particles has been demonstrated in the single receptor immunosuppressive drug assay format for detecting rapamycin in co-pending and commonly assigned US 60/714,712 filed September 7, 2005 and US 11/468,940 filed August 31, 2006, the content of which applications are herein incorporated by reference. The use of nanoparticles is also disclosed therein.

Armstrong et al., in Clinical Chemistry 44:12, 2516-2523 (1998) describe a pentamer formation assay for measurement of tacrolimus and its active metabolites after extraction from whole blood. They describe a microtiter plate-based assay using binding proteins.

Sagi-Eisenberg, in WO 2005/062708 published July 14, 2005, teaches a heterogeneous assay for the detection ofrapamycin utilizing immobilized FKBP12.

Huster, in US 2006/0099654 published May 11, 2006, describes an immunoassay method for determining sirolimus by combining a sample suspected of containing sirolimus with two latex bead reagents and a biotinylated analyte receptor. One bead reagent is coated with streptavidin and contains a photosensitive dye. A second bead reagent is coated with an antibody generated using a fragment of the sirolimus molecule and contains a chemiluminescent dye. The analyte receptor can be FKBP.

EP 1239285 discloses an homogeneous one receptor assay for detecting an analyte in a sample using antibody specific for analyte, biotinylated analyte and streptavidin latex particles. When analyte successfully competes with analyte-biotin complex, the there are less complexes formed which induces further aggregation of the particle complexes. EP 1239285 does not describe an homogeneous double receptor agglutination assay for immunosuppressant drugs.

WO 2005/088307 discloses an homogeneous microparticle (immunoturbidimetric immunoassay) for everolimus using antibody specific for analyte, biotinylated analyte and streptavidin-latex particles. When analyte successfully competes with analyte-biotin complex, then there are less complexes formed which induces further aggregation of the particle complexes. There is no involvement of 2 receptors in the agglutination assay.

US 6187547 an homogeneous assay method of measuring blood levels of immunophilin-binding pharmaceuticals (FK506 or rapamycin, cyclosporins) involving the step of displacing the pharmaceutical from its immunophilin by using a binding competitor. The receptor consequently binds the pharmaceutical. No particles are involved in said method that could allow agglutination.

US 6338946 describes a method of assaying immunosuppressants having calcineurin-inhibiting activity comprising the steps of forming a complex with FKBP12 bound to solid phase, calcineurin, calmodulin, calcium ion and immunosuppressant. This method is not an agglutination assay.

WO 2007/028580 (an Article 54(3) EPC document) describes a homogeneous assay method for immunosuppressive drug using an immunophilin in a single receptor format. Thereby, an immunophilin is substituted for an antibody, and a competition results between a drug conjugate and the drug analyte for a limited number of immunophilin binding sites.

WO 2006/108130 (an Article 54(3) EPC document) provides a method to determine levels of immunosuppressive complexes containing the immunosuppressive drugs tacrolimus, sirolimus and cyclosporine A separately and in combination, formed in the blood of a drug-treated patient or a patient candidate to immunosuppressive drug therapy.

### Summary of the invention

The present invention comprises a method for determining the presence or amount of an immunosuppressive drug in a sample comprising the steps of (a) providing a sample suspected of containing the immunosuppressive drug, (b) adding to the sample at least two receptors that are specific for the drug, wherein each of the receptors is specific for a separate binding site on the drug and wherein each of the receptors is bound to a detection particle, and further wherein the drug binds to the receptors and causes particle agglutination, (c) measuring the amount of particle agglutination, and (d) correlating the amount of particle agglutination with the presence or amount of immunosuppressive drug in the sample. In accordance with one embodiment, only one of the receptors is bound to a detection particle, and an antibody that specifically binds to the receptor protein not bound to a particle is used to agglutinate the particles. In another embodiment an antibody specific for the target immunosuppressant drug is substituted for one or both of the two receptors, wherein the antibody is bound to a detection particle.

### Brief description of the drawings

Figure 1. Rapamycin calibration curve according to the present invention as described in Example 8 using yTOR1 FRBD. Rapamycin concentration in spiked whole blood samples is plotted on the x-axis and absorbance is plotted on the y-axis.
Figure 2. Rapamycin calibration curve according to the present invention as described in Example 8 using yTOR2 FRBD. Rapamycin concentration in spiked whole blood samples is plotted on the x-axis and absorbance is plotted on the y-axis.
Figure 3. Chart comparing the measured rapamycin concentration (y-axis) vs. the spiked rapamycin concentration (x-axis) according to the present invention as described in Example 8. Rapamycin concentration in spiked whole blood samples is plotted on the x-axis and the measured rapamycin concentration is plotted on the y-axis.
Figure 4. Rapamycin calibration curve according to the present invention as described in Example 15 using mTOR FRBD. Rapamycin concentration in spiked whole blood samples is plotted on the x-axis and absorbance is plotted on the y-axis.
Figure 5. Plot showing interactions of FK506-bound FKBP12 with truncated calcineurin A/B fusions.

### Detailed description of the invention

As used herein, the term "detection particle" refers to any particle having a generally spherical shape with a diameter ranging from about 10 nm to about 1000 nm that allows one to distinguish between an aggregated state and a non-aggregated state in a homogeneous assay. The detection particle can be a nanoparticle or a microparticle, but for the purposes of convenience, all such particles are referred to herein as "microparticles". The surface of the detection particle may be functionalized, and various compounds may be attached to the surface, including for example, fluorophores, chemiluminescent entities, antibodies, biotin, or streptavidin.

An epitope is an area on the surface of an antigenic molecule that stimulates a specific immune response and against which that response is directed. Epitope tagging is a recombinant DNA method by which a protein encoded by a cloned gene is made immunoreactive to a known antibody. Development of fusion tag systems provides great flexibility for easy purification and detection of recombinant proteins using affinity matrices and specific antibodies. Epitope tags range from 10 to 15 amino acids in length and are designed to create a molecular handle for a protein. They are typically placed on either the amino or carboxyl terminus to minimize any potential disruption in tertiary structure and thus function of the protein. Because the tags are small, they are further unlikely to interfere with structure and function of the recombinant protein. Therefore, an epitope tag does not usually need to be removed before subsequent experiments are performed. A number of different tags have been used, for example, His₆ (a sequence of six histidines that have a strong affinity for matrices containing metal ions like Ni⁺² or Co⁺²), HA (a peptide sequence derived from the human influenza virus hemagglutinin protein), and AviTag, a sequence which can be enzymatically mono-biotinylated at its internal lysine residue by *E*. *coli* biotin protein ligase (BirA). The enzyme BirA can be added to the expression mix and the biotinylation performed *in situ.*

The term "FK506 binding protein", or "FKBP", as used herein refers to a receptor protein that binds to rapamycin to form a complex that will specifically bind to a protein selected from the group consisting of mTOR, yTOR1, yTOR2, and the FRBDs of mTOR, yTOR1, and yTOR2.

The term "specific binding" refers to a high avidity and/or high affinity binding between two paired species, including for example, such paired species as ligand/target moiety, enzyme/substrate, receptor/agonist, antibody/antigen, and lectin/carbohydrate. The binding interaction may be mediated by covalent or non-covalent interactions or a combination of covalent and non-covalent interactions. In particular, the specific binding is characterized by the binding of one member of a pair to a particular species and to no other species within the family of compounds to which the corresponding member of the binding member belongs. Thus, for example, an antibody preferably binds to a single epitope and to no other epitope within the family of proteins.

The term "receptor" or "binding moiety" as used herein, refers to a protein or glycoprotein that specifically binds to a target compound. An example of a receptor is an immunophilin that binds to an immunosuppressant drug.

The term "nanoshell" as used herein refers to a microparticle composed of a dielectric core (for example, silica or calcium phosphate) coated with an ultra-thin metallic layer (for example, gold). Nanoshells suitable for use in the present invention have a diameter selected from a range of about 50 nm to about 250 nm. The surface of the microparticle may be functionalized, and various compounds may be attached to the surface, including for example, antibodies or streptavidin.

The term "microparticle" as used herein refers to a solid particle of about 10 nm to about 1000 nm in diameter. Typically for use in the present invention, the microparticles will have a diameter of about 100 nm to about 600 nm. Microparticles can be formed from a variety of materials and include polystyrene and latex microparticles, the surfaces of which are optionally functionalized, and various compounds may be attached to the surface, including, for example, antibodies or streptavidin.

As used herein, the term "antibody" refers to a polypeptide or group of polypeptides which are comprised of at least one binding domain, where an antibody binding domain is formed from the folding of variable domains of an antibody molecule to form three-dimensional binding spaces with an internal surface shape and charge distribution complementary to the features of an antigenic determinant of an antigen, which allows an immunological reaction with the antigen. Unless otherwise stated a general reference to an antibody encompasses polyclonal as well as monoclonal antibodies. The term "antibody" also includes recombinant proteins comprising the binding domains, as well as fragments of antibodies, including Fab, Fab', F(ab)₂, and F(ab')₂ fragments.

As used herein a "linker" is a bond, molecule, or group of molecules that binds two separate entities to one another. Linkers may provide for optimal spacing of the two entities or may further supply a labile linkage that allows the two entities to be separated from each other. Labile linkages include photocleavable groups, acid-labile moieties, base-labile moieties, and enzyme-cleavable groups.

As used herein the term "homogeneous assay" refers to a quantitative or qualitative test for determining the presence or concentration of a compound in a sample, wherein the unreacted test reagents do not need to be separated from the reagents that have reacted with the target compound in order to detect, or measure the concentration of, the target compound.

As used herein the term "sandwich assay" refers to a homogeneous assay for detecting a target compound wherein the assay utilizes two binding moieties that each specifically bind to the target compound but at two separate locations on the target compound.

The present invention relates to a homogeneous, particle-based, sandwich assay to detect an immunosuppressant drug in a biological fluid. More particularly, the present invention relates to a homogeneous particle-based assay using binding moieties that specifically bind to immunosuppressant drugs. The assay comprises at least two binding moieties, wherein each of the separate binding moieties binds to a different location on the target immunosuppressant drug, and each of the binding moieties is bound to one or more detection particles. Accordingly, during the assay a sample suspected of containing the immunosuppressant drug of interest is contacted with a composition comprising two binding moieties such that the degree of agglutination of the reactants, i.e., the number of sandwich complexes formed between the two binding moieties and the immunosuppressant drug, indicates the amount of immunosuppressant drug present in the sample.

Detecting the degree of agglutination is done using a homogeneous assay format wherein the unreacted reagents, i.e., the unbound binding moieties, do not need to be separated from the reacted reagents (i.e., sandwich complexes comprising two binding moieties and the immunosuppressant drug). Homogeneous assay systems are known to those skilled in the art and can be adapted for use in the present invention.

In accordance with one embodiment, the two binding moieties are bound to detection particles and the step of measuring the amount of agglutination occurring upon contact of a sample with the two binding moieties is conducted by measuring the turbidity of the sample relative to a reference. The effect on the degree of agglutination can be determined by absorbance measurement. In accordance with one embodiment the detection particle is a microparticle, and a microparticle agglutination assay is employed to determine the presence or quantity of an immunosuppressant drug in a sample. Such microparticle agglutination assays are considered to be homogeneous assays because a washing step is not employed prior to the step of measuring the amount of agglutination that has occurred. Microparticle agglutination assays are conveniently performed and measured using automated analyzers such as the Roche/Hitachi and COBAS analyzers (Roche Diagnostics, Indianapolis). In the microparticle agglutination assay of the present invention, a first binding moiety and a second binding moiety are bound, directly or indirectly, to microparticles. The first and second binding moieties are specific for different binding sites on the drug to be determined. In a reaction solution, these reagents (binding moieties bound to microparticles) are dispersed in the liquid medium and form a stable suspension. In the presence of the immunosuppressant drug, binding between the drug and the receptors (binding moieties) occurs, causing particle agglutination and a corresponding increase in turbidity of the solution, which is quantified by measuring absorbance of the solution. There is a direct correlation between the amount of drug present in a sample and the amount of absorbance measured.

In accordance with one embodiment the detection particle is a nanoshell. Nanoshells are a type of optically tunable detection particle composed of a dielectric (for example, silica, alginate, or calcium phosphate) core coated with an ultra-thin metallic (for example, gold) layer. Gold nanoshells possess physical properties similar to gold colloid, in particular a strong optical absorption due to the collective electronic response of the metal to light. The optical response of gold nanoshells depends dramatically on the relative sizes of the nanoparticle core and the thickness of the gold shell. By varying the relative core and shell thicknesses, the color of gold nanoshells can be varied across a broad range of the optical spectrum that spans the visible and the near-IR spectral regions. Gold nanoshells can be made either to absorb or scatter light preferentially by varying the size of the particle relative to the wavelength of the light at their optical resonance. In one embodiment the nanoshell core has a diameter selected from the range of about 50 nm to about 200 nm, and in one embodiment about 120 nm, and the thickness of the nanoshell coating or shell is selected from a range of about 5 nm to about 30 nm.

A whole blood immunoassay has been described using gold nanoshells (Anal. Chem. 75:2377-2381, 2003). It is anticipated that binding proteins such as immunophilins could be substituted for antibodies in the nanoparticle assay described. The principle of nanoparticle aggregation is similar to that of microparticle agglutination technique. Blood's high turbidity and strong visible extinction complicate the detection of aggregates in an agglutination assay. However selection of the optimal wavelengths and pretreatments of a blood sample can overcome these complicating factors. Nanoshells also are advantageous in that they are quite stable with respect to aggregation in the whole blood and dimerization or aggregation of the nanoparticles by a sandwich assay format (immunosuppressive drug with binding proteins) can be detected via spectral red-shifting in the near infra-red, where whole blood is more transmissive, thus allowing detection of nanoshell aggregation in whole blood.

In accordance with one embodiment an assay method for determining a presence or an amount of an immunosuppressant drug in a sample is disclosed comprising the steps of providing a sample; mixing said sample with a first receptor and a second receptor to form a suspension, wherein said first and second receptors are bound to detection particles and each of said first and second receptors specifically bind to a separate binding site on said drug, wherein the presence of the drug results in agglutination of the detection particles; directly detecting or measuring an amount of particle agglutination in said suspension; and correlating the amount of particle agglutination with the presence or amount of the immunosuppressant drug in the sample. A further embodiment refers to the immunosuppressant drug is selected from the group consisting of cyclosporin, tacrolimus, rapamycin, and everolimus, and said first receptor is an immunophilin or a binding fragment thereof. A further embodiment refers to the detection particles are selected from the group consisting of microparticles and nanoshells.

In accordance with one embodiment the immunosuppressant drug to be detected and quantitated is selected from the group consisting of cyclosporin, tacrolimus, rapamycin, and everolimus, and the binding moieties are immunosuppressant drug binding receptor proteins. In one embodiment the receptors represents native immunophilins of the target immunosuppressant drug, or binding fragments or derivatives of said native immunophilins. In one embodiment the target the immunosuppressant drug is rapamycin or everolimus and the first receptor comprises a FK506 binding protein (FKBP) and the second receptor comprises a target of rapamycin (TOR) protein. In a further embodiment the FKBP is FKBP12 or FKBP25 and the TOR is selected from the group consisting of mTOR, yTOR1, yTOR2, and the FRBDs of mTOR, yTOR1, and yTOR2.

In an alternative embodiment the immunosuppressant drug to be detected and quantitated in a sample is tacrolimus, wherein the first receptor comprises an FK506 binding protein (FKBP) and the second receptor comprises calcineurin, or a binding fragment or derivative thereof. In a further embodiment the second receptor further comprises calmodulin and a calcium ion bound to calcineurin. In yet another embodiment wherein the immunosuppressant drug to be detected and quantitated is a cyclosporin, wherein the first receptor comprises a cyclophilin selected from the group consisting of cyclophilin A (CypA), cyclophilin B (CypB), cyclophilin C (CypC), the structures of which are described in Mikol, et al. PNAS 91:5183-5186 (1994), and the second receptor comprises an calcineurin. In a further embodiment the second receptor further comprises calmodulin and a calcium ion bound to calcineurin.

In accordance with one embodiment the immunosuppressant drug binding receptor protein is bound to a detection particle wherein the detection particle is a nanoshell or microparticle. The detection particle can be bound to the receptor protein using any of the standard techniques known to those skilled in the art. For example the detection particle can be bound to the receptor protein through a direct covalent linkage using standard linkers or the protein can be bound to the detection particle through ionic, hydrogen bonding, hydrophobic/hydrophilic interactions or any combination thereof. In a one embodiment of the invention, receptor proteins are attached to the detection particle via direct coupling or through a tag.

As one example, detection particles can be attached to anti-tag antibodies and binding proteins can be attached to the corresponding tag. Alternatively, a first receptor protein can be attached to a tag (hapten), including for example biotin, and the detection particles can be bound to streptavidin or an antibody that binds to the tag (hapten). The second binding protein of the drug is also attached to the same tag or in an alternative embodiment to a different tag. The number of biotin or tagged molecules attached to the protein can be varied, but most preferably one biotin (or other tag) will be attached per molecule of binding protein to minimize spontaneous agglutination of the streptavidin microparticles (or ligand for said tag).

In accordance with one embodiment, streptavidin, avidin or an anti-biotin antibody (or an anti-hapten antibody) is attached to the detection particle. In the presence of immunosuppressant drug, a sandwich is formed simultaneously with the drug and two biotinylated binding proteins. In the presence of a streptavidin labeled detection particle, agglutination occurs, which can be measured by light scattering. The more drug present in the sample, the more agglutination is observed. When no drug is present, no sandwich is formed and the detection particles will not agglutinate. In accordance with one embodiment the detection particle is a streptavidin labeled microparticle and the biotinylated receptor proteins are pre-attached to the streptavidin microparticle prior to contacting the receptor proteins with the sample comprising the immunosuppressant drug. This procedure provides the most optimal results in the assay.

In a similar manner when the two receptor proteins are labeled with one member of a fluorescence resonance energy transfer (FRET) fluorophore pair, respectively the presence of the immunosuppressant drug can be detected. In the presence of the immunosuppressant drug, a sandwich is formed simultaneously with the drug and two biotinylated binding proteins. The formation of this complex brings the two fluorophores within sufficient-proximity to one another that upon excitation of the donor fluorophore with excitation light, fluorescent energy transfer occurs. Such an event can be measured by detecting a decrease in donor fluorophore emissions or an increase in the acceptor fluorophore's emissions.

In accordance with one embodiment the detection particles are bound to said first and second receptors via a biotin-streptavidin or biotin-avidin linkage. In a further embodiment the detection particles are covalently bonded either directly or indirectly through a linker to said first and second receptors. In addition, a linker comprises an antibody to the first receptor and an antibody to the second receptor. The first and second receptors are recombinant fusion proteins comprising an attachment site for a molecule of biotin.

In accordance with one embodiment the receptor proteins can be expressed as recombinant fusion proteins that include a peptide sequence that either represents a ligand or tag or provides an optimal site for attachment of a molecule of biotin. For example the receptor protein can be a recombinant protein that comprises a fusion partner with a biotinylation signal sequence. This fusion protein is co-expressed in *Escherichia coli* or in a cell-free system with biotin ligase, e.g., AviTag, which catalyses the covalent addition of a biotin moiety to a lysine residue in the signal sequence. In one embodiment the biotin ligase comprises AviTag and the residue is lysine. In one embodiment the two different receptor proteins used in the assay are AviTagged biotin labeled proteins. The AviTagged sequence can be attached through either the C-terminus or N-terminus. Models of the sandwich concept seem to suggest that at least one of the tags should be placed at the N-terminus to prevent steric hindrance. However, applicants have surprisingly found that the assay works well with both tags at the C-terminus.

In an alternative embodiment, antibodies to the receptor protein are bound to the surface of the detection particles, wherein the bound antibodies specifically bind to the receptor protein itself or to a tag that is bound to the receptor protein. In the presence of immunosuppressant drugs the sandwich forms and agglutination occurs. These antibodies are designed and or selected in such a way that the antibodies do not bind to the receptor protein binding site for the immunosuppressant drugs.

In accordance with one embodiment assay reagents are provided for detecting and quantifying the amount of an immunosuppressant drug present in a sample. The sample may be any liquid sample that is suspected of containing an immunosuppressant drug. More typically the sample is a biological bodily fluid recovered from a patient being administered an immunosuppressant drug. In one embodiment the sample is whole blood or a derivative product thereof. When whole blood is used as the sample and the assay is a microparticle agglutination detection method, the blood sample is typically pretreated prior to contacting the sample with the assay reagents in order to release cellular-bound drugs. This procedure includes combining the whole blood sample with a precipitating agent. The precipitating agent is selected from those known in the art including, for example, aqueous copper sulfate or zinc sulfate in methanol, ethanol, ethylene glycol, acetonitrile or similar water miscible organic solvents. The whole blood mixture is then mixed thoroughly and centrifuged. The clear supernatant is then transferred into the sample cup and placed on the analyzer for assay performance. In one embodiment the composition used to pretreat the whole blood sample comprises methanol and zinc sulfate. After pretreating the sample, the cellular debris is removed prior to adding the assay reagents. In one embodiment the cellular debris is removed by centrifugation at a low speed (approximately 12,000 rpm) for 5 minutes.

The assay reagents in accordance with one embodiment comprise a first receptor and a second receptor wherein the first and second receptors specifically bind to separate binding sites on the target drug to form a sandwich complex upon contact of the reagents with the drug. In accordance with one embodiment, the first and second receptors are each provided with a tag for binding the receptor to a detection particle. In another embodiment the first and second receptors will be provided with the detection particle already bound to the first and second receptors. In one embodiment the first and second receptors further comprise a biotinylated tag and the detection particle is bound to streptavidin. In a further embodiment each of the first and second receptors is provided with a single biotin molecule. In one embodiment the detection particle is a microparticle bound to streptavidin or to an antibody specific for biotin. In one embodiment the immunoassay reagents are provided as two complexes comprising a receptor bound to a microparticle through a biotin-streptavidin linkage.

In one embodiment a reagent is provided for determining an immunosuppressant drug in a sample, said reagent comprising a first drug binding complex comprising a first receptor bound to a first detection particle;a second drug binding complex comprising a binding moiety selected from the group consisting of a second receptor bound to a detection particle, an immunosuppressant drug specific antibody bound to a second detection particle, and a second receptor specific antibody complexed with a second receptor, wherein each drug binding complex specifically binds to a separate binding site on said immunosuppressant drug. In a further embodiment the binding moiety is a second receptor bound to a detection particle.

In one embodiment an agglutination assay reagent is provided for quantifying the amount of rapamycin or everolimus in a sample. In this embodiment, the first receptor comprises an FK506 binding protein bound to a detection particle and said second receptor comprises a target of rapamycin (TOR) protein bound to a detection particle. In one embodiment a reagent is provided wherein the detection particle is a microparticle. In a further embodiment-a reagent is provided wherein the second drug binding complex comprises a second receptor specific antibody complexed with a second receptor. In one embodiment the first receptor comprises FKBP12 or FKBP25 and the second receptor is selected from the group consisting of mTOR, yTOR1, yTOR2, and the FRBDs of mTOR, yTOR1, and yTOR2. In an alternative embodiment an agglutination assay reagent is provided for quantitating the amount of tacrolimus present in a sample, wherein the reagents comprise a first receptor comprising an FK506 binding protein (FKBP) bound to a detection particle and a second receptor comprising calcineurin, or a binding fragment or derivative thereof, bound to a detection particle. In a further embodiment the second receptor further comprises calmodulin and a calcium ion bound to calcineurin. In yet another embodiment an agglutination assay reagent is provided for quantitating the amount of cyclosporin, wherein the reagents comprise a first receptor comprising a cyclophilin bound to a detection particle selected from the group consisting of cyclophilin A (CypA), cyclophilin B (CypB, and cyclophilin C (CypC), and a second receptor comprising a calcineurin or fragment of calcineurin bound to a detection particle. In a further embodiment the second receptor further comprises calmodulin and a calcium ion bound to calcineurin. The detection particle is typically either a nanoshell or a microparticle.

In accordance with one embodiment a method is provided for determining the amount of an immunosuppressant drug in a sample. The method comprises the steps of isolating a sample, and optionally pretreating the sample to release the cellular bound immunosuppressant drug. The sample is then contacted with an assay reagent composition comprising a first and second receptor to form a suspension, wherein the first and second receptors specifically bind to separate locations on the target immunosuppressant drug. In one embodiment, the reagent composition will further comprise detection particles that bind to the first and second receptors. Alternatively, in one embodiment the detection particles are provided as a separate component that is added to the suspension before, during or after the addition of the first and second receptors. The assay reagents are mixed together, to induce agglutination in the presence of the target drug, wherein the first and second receptors each specifically bind to a separate binding site on said drug and the detection particles bind each of the receptors (if not already bound to the receptors) to form a detectable sandwich complex. The amount of agglutination is then measured directly, meaning that no further purification or washing steps are required, and thus the assay is a homogeneous assay. The amount of immunosuppressant drug is correlated to the detected amount of particle agglutination, using homogeneous assay techniques known to those skilled in the art. In accordance with one embodiment the extent of particle agglutination is measured by an absorbance measurement and the amount of drug present is determined by reference to a standard curve.

In one embodiment the a method for determining a presence or an amount of an immunosuppressant drug in a sample is disclosed comprising the steps of providing a sample; mixing said sample with a receptor bound to a first detection particle and a binding moiety selected from the group consisting of an immunosuppressant drug specific antibody bound to a second detection particle, and a second receptor specific antibody complexed with a second receptor to form a suspension, wherein said receptor specifically binds to said immunosuppressant drug at a location different from where the immunosuppressant drug specific antibody binds, wherein the presence of the drug results in agglutination of the detection particles; measuring an amount of particle agglutination in said suspension by absorbance measurement; and correlating the amount of particle agglutination with the presence or amount of the immunosuppressant drug in the sample. In a further embodiment the immunosuppressant drug is selected from the group consisting of cyclosporin, tacrolimus, rapamycin, and everolimus and the binding moiety is an antibody specific for the immunosuppressant drug, wherein the antibody is bound to a detection particle. In a further embodiment the immunosuppressant drug is rapamycin or everolimus; the first receptor comprises an FK506 binding protein (FKBP); and the binding moiety comprises a target of rapamycin (TOR) protein or antibody that specifically binds to a target of rapamycin (TOR) protein.In a further embodiement the immunosuppressant drug is tacrolimus; the first receptor comprises an FK506 binding protein (FKBP); and the second receptor comprises a calcineurin or antibody that specifically binds to calcineurin. In a further embodiment the the immunosuppressant drug is cyclosporin; the first receptor comprises a cyclophilin; and the second receptor comprises a calcineurin or antibody that specifically binds to calcineurin.

In one embodiment the detection particles are either microparticles or nanoshells, and in one embodiment the detection particles are microparticles. The method can be used to measure the amount of an immunosuppressant drug selected from the group consisting of cyclosporin, tacrolimus, rapamycin, and everolimus by selection of the receptors used in the assay. To detect rapamycin or everolimus, the first receptor comprises a FK506 binding protein (FKBP) bound to a first detection particle and the second receptor comprises a target of rapamycin (TOR) protein bound to a second detection particle. To detect tacrolimus, the first receptor comprises an FK506 binding protein (FKBP) bound to a first detection particle, and the second receptor comprises a calcineurin bound to a second detection particle. To detect cyclosporine the first receptor comprises a cyclophilin bound to a first detection particle, and the second receptor comprises an calcineurin bound to a second detection particle. For methods directed to detecting and quantitating the presence of tacrolimus or cyclosporine the second receptor (calcineurin) may optionally be further complexed with calmodulin and a calcium ion.

The method for quantitating an immunosuppressant drug can be conducted on any aqueous sample that is believed to contain an immunosuppressant drug as a means of confirming the presence and determining the concentration of the drug in that sample. In one embodiment the sample is a bodily fluid isolated from a patient, including such fluids as blood, plasma, serum, urine, sperm, cerebral spinal fluid, saliva and the like. In one embodiment the sample is whole blood. Typically the blood sample is pretreated to release the cellular bound immunosuppressant drug before the sample is contacted with the assay reagents. In one embodiment the blood sample is extracted with a solution comprising methanol and zinc sulfate and cellular debris is removed prior to said mixing step. In one embodiment the detection particles are nanoshells and the blood sample is not pre-treated prior to the addition of the assay reagents.

In one embodiment, anti-immunosuppressant drug antibodies can also be used in an agglutination assay in place of one or both receptor proteins.

Antibodies (and in one embodiment monoclonal antibodies) can be raised against a complex comprising an immunosuppressant drug and an immunophilin, wherein the antibody is selected based on its binding to the immunosuppressant drug. In this manner the specificity of the antibody for active immunosuppressant drugs may be enhanced relative to inactive metabolites of the immunosuppressant drug. As an example, an antibody could be raised against a portion of the rapamycin molecule specific to FKBP12 binding site (wherein the immunogen used comprises a complex of rapamycin and a target of rapamycin (TOR) protein). Alternatively, an antibody could be raised against a portion of the rapamycin molecule specific to FKBP-rapamycin associated protein binding site (wherein the immunogen used comprises a complex of rapamycin and FKBP12). A similar approach would be applicable to other immunosuppressant drugs including cyclosporin, tacrolimus, and everolimus. For example, an antibody is raised against FK-506 bound to FKBP12 and used to measure FK-506 in a sample. The binding of FK-506 is very tight to FKBP12 and the FK-506/FKBP12 complex may be coupled to KLH as an immunogen to be injected into mice to elicit an immune response. (In this case the coupling would be done between FKBP12 and KLH through standard protein-protein coupling methods.) The isolation of suitable antibodies and selection of monoclonal antibodies would be done using standard methods known in the art. This approach can also be used to produce antibodies against cyclosporin A bound to cyclophilin.

In accordance with one embodiment a method for determining the amount of an immunosuppressant drug in a sample is provided, wherein the assay reagents are selected from the group consisting of receptor proteins bound to detection particles and antibodies bound to detection particles. In one embodiment the antibodies are monoclonal antibodies. In one embodiment the assay is conducted with two antibodies wherein the antibodies were raised against an immunosuppressant drug/immunophilin complex, but having specificity for two different sites on the immunosuppressant drug. In this embodiment the two antibodies are coupled to a detection particle. In another embodiment the assay is conducted with two antibodies wherein the antibodies were raised against an immunosuppressant drug/immunophilin complex, but having specificity for two different sites on the immunophilin. In this embodiment the two antibodies are coupled to a detection particle. In a further embodiment the assay reagents may comprise one receptor that specifically binds to the target immunosuppressant drug and one antibody that specifically binds to a different location of the immunosuppressant drug. In this embodiment both the receptor protein and the antibody are bound to detection particles.

In a further embodiment the assay reagents may comprise a first receptor, second receptor and an antibody that specifically binds to the second receptor. In this embodiment, the first receptor is bound to a detection particle, and the first receptor specifically binds to the target immunosuppressant drug. The second receptor protein binds to the target immunosuppressant drug at a different location than the first receptor protein. In this embodiment the second receptor protein is not bound to a detection particle and the antibody is typically not labeled. However the antibody may be optionally bound to a detection particle. In one embodiment the antibody that specifically binds to the second receptor protein is generated against an immunogen comprising a second receptor/immunosuppressant drug complex. In one of the embodiments, one of the receptors, e.g., FKBP12 (for rapamycin) is labeled with a tag, e.g., biotin. When a sample is mixed with a limited amount of biotinylated first receptor protein and a second receptor protein, a sandwich complex is formed with the target drug present in the sample. The resultant biotinylated sandwich complex plus unreacted FKBP12 is then added to a second reagent containing streptavidin latex microparticles and an antibody which specifically binds to the second receptor protein. Agglutination takes place in proportion to the number of biotinylated sandwich complexes bound to streptavidin latex and agglutinated by the antibody, which in turn is directly related to the amount of drug present. Antibody reacting with free non-complexed second receptor partner does not result in any signal. It is important to note that in this alternative, the antibody binds to the second receptor at a site other than the receptor binding site for the drug. Anti immunophilin antibodies that specifically bind to an FKBP/rapamycin-binding domain are described in US 6,464,974.

In accordance with one embodiment a method of quantitating an immunosuppressant drug comprises providing a sample and mixing the sample with a reagent composition to form a suspension. The reagent composition comprises a receptor and an antibody, each of which is bound to a detection particle and wherein each of said receptor and an antibody specifically and simultaneously bind to the immunosuppressant drug. The amount of particle agglutination present in the suspension is then measured using a homogeneous assay technique, such as absorbance measurement, for example, and the amount of detected particle agglutination is then correlated with the amount of immunosuppressive drug present in the sample.

In accordance with another embodiment a method of quantitating an immunosuppressant drug comprises providing a sample, mixing the sample with an reagent composition to form a suspension, wherein the reagent composition comprises a first and second antibody, each of which is bound to a detection particle and wherein each of said first and second antibody specifically and simultaneously bind to the immunosuppressant drug. The amount of particle agglutination present in the suspension is then measured using a homogeneous assay technique, such as by absorbance measurement, for example, and the amount of detected particle agglutination is then correlated with the amount of immunosuppressive drug present in the sample.

In accordance with another embodiment a method of quantitating an immunosuppressant drug comprises providing a sample, mixing the sample with an reagent composition to form a suspension, wherein the reagent composition comprises a first and second receptor and antibody that specifically binds to the second receptor, wherein each of said first and second receptors specifically and simultaneously bind to the immunosuppressant drug, and the first receptor is bound to a detection particle. In one embodiment the antibody is also bound to a detection particle. The amount of particle agglutination present in the suspension is then measured using a homogeneous assay technique, such as by absorbance measurement, for example, and the amount of detected particle agglutination is then correlated with the amount of immunosuppressive drug present in the sample.

The present invention also anticipates the use of receptor mutants that have enhanced selectivity for the parent drug. In accordance with one embodiment yTOR2 FRBD and FKBP12 peptide mutants are generated using site-directed mutagenesis in order to enhance the specificity of the assay for the parent drug of rapamycin. The concept to mutate the receptor proteins in order to enhance the specificity of the receptor complex for the parent drug of rapamycin could be applied to any of the receptor proteins disclosed herein. The formation of the yTOR2 FRBD/rapamycin/FKBP12 complex can be monitored by several of the methods described herein and used to measure the rapamycin concentration in patient samples.

Publications of crystal structures can be used as an aid in the mutagenesis studies. The mutant binding proteins can be produced through recombinant methods well-known in the art and have affinity tags added through recombinant methods to aid in their purification, using methods that are also well known in the art. Accordingly, with reference to the following table, it is anticipated that the following amino acid residues can be modified to enhance the specificity of the molecules: the glutamic acid in position 54 of FKBP12, the asparagine in position 2038 of yTOR2, the aspartic acid in position 1972 of yTOR2, and the arginine in position 1976 of yTOR2.

The following table shows C-C distances measured between rapamycin metabolites, FKBP12 and FRBD. The crystal structure 1fap published by the RCSB PDB (Protein Data Base) was used to measure the C-C distances of the rapamycin metabolites (12).

| | PDB 1fap C-C distances | C moiety | FKBP12 (Å) | FRB (Å) | TOR2 |
|---|---|---|---|---|---|
| M1 | 12-hydroxy-Rapa | C12 | H87 (7.12) I90 (6.90) | T2098 (7.07) | N2038 |
| M2 | 24-hydroxy-Rapa | C24 | E54 (7.69) | L2031(6.53) | L1971 |
| | | | | E2032 (4.67) | D1972 |
| M3 | 16-O-desmethyl-Rapa | C50 | | T2098 (4.00) | N2038 |
| | | | | Q2099 (5.73) | Q2039 |
| | | | | D2102 (4.04) | D2042 |
| M4 | 39-O-desmethyl-Rapa | C52 | E54 (6.79) | R2036 (4.54) | R1976 |
| | | | | G2040 (3.93) | G1980 |
| M5 | 27, 39-O-didesmethyl-Rapa | C51, | | E2032 (5.18) | D1972 |
| | | (C52 see M4) | | E2033 (6.48) | D1973 |
| | | | | S2035 (5.45) | S1975 |
| | | | | R2036(4.18) | R1976 |

The detection of complex formation can be conducted using a fluorescence resonance energy transfer (FRET) pair of fluorophores but is not part of the present invention. FRET is based upon the distance-dependent transfer of excited-state energy from a donor fluorophore to an acceptor fluorophore. The donor fluorophore is excited by incident light and if an acceptor is within 20Å to 60Å, the excited state energy from the donor can be transferred. Beyond the optimum range of intermolecular distances, the energy transfer efficiency falls off as the inverse sixth power of the distance. This transfer leads to a reduction in the donor's fluorescence intensity and excited-state lifetime, and an increase in the acceptor's emission intensity (Selvin, P.R., Nature Structural Biology, 2000, 7, 9, 730-734). This assay format requires that a pair of FRET fluorophores be coupled to a pair of binding receptors. A donor fluorophore such as europium and an acceptor fluorophore such as Cy5 have been used using light excitation at 340 nm and emission detection at 665 nm (Pulli, T., et. al., Analytical Chemistry, 2005, 77, 2637-2642). Accordingly, first and second receptor proteins that specifically bind to an immunosuppressant drug can be labeled with the respective donor and acceptor fluorophores. For example, in an assay for detecting rapamycin, an europium donor fluorophore can be used to label a first receptor (for example, FKBP12) and a Cy5 acceptor fluorophore can be used to label a second receptor (for example yTOR2 FRBD). The distances measured between selected amino acids in the mTOR FRBD-rapamycin-FKBP12 complex (1fap, Protein Data Bank) are listed in the table below. One possible combination is to label the amino (NH₂) terminus of yTOR2(R1959) and label the amino terminus of FKBP12 (G1) which are 48.56 Å apart in the crystal structure, assuming that yTOR2 adopts a conformation similar to mTOR FRBD in the mTOR FRBD-rapamycin-FKBP12 complex.

| | mTOR FRBD (yTOR2) | Distance | FKBP12 |
|---|---|---|---|
| amino acid position | R2018(R1959)(NH₂ terminus) | 38.5 Å | E107 (COOH terminus) |
| | R2018 (R1959)(NH₂ terminus) | 48.56 Å | G1 (NH₂ terminus) |
| | S2112 (G2052)(COOH terminus) | 32.97 Å | G1 (NH₂ terminus) |

Several commercial kits are available to label the amino acids of yTOR2 and FKBP12 with the donor and acceptor fluorophores. For example, the LANCE Eu-W 1024-ITC chelate (Perkin Elmer, AD0013) is optimized for the covalent labeling of proteins possessing at lease one primary aliphatic amine (N-terminus or lysine residues) with europium. Additionally, the CyDye Cy5 mono-Reactive Dye pack (Amersham, Product Code PA23500) can be used for the covalent labeling of amine groups on proteins with Cry5. Other chemistries are available to enable labeling via amine groups (using NHS ester dyes), thiol groups (using maleimide dyes), or aldehyde groups (using hydrazide dyes). The yTOR2 and FKBP12 receptors can be labeled accordingly and the unlabeled fluorophores can be removed from the labeled receptors by column chromatography.

The assay can be performed by the addition of rapamycin to appropriately labeled yTOR2 and labeled FKBP12. The presence of rapamycin will drive the formation of the yTOR2/rapamycin/FKBP12 complex in a rapamycin dependent manner. Using a fluorometer one can measure the fluorescence resulting from the light excitation of the yTOR2/rapamycin/FKBP12 complex formed. In this example, the fluorescence measured is directly proportional to the rapamycin concentration. One can construct a standard curve with known concentrations of rapamycin. The unknown rapamycin concentration in patient samples can thus be calculated based on the standard curve. Similarly, other immunosuppressant drugs can be detected by labeling the receptor proteins FKBP12 and calcineurin for detecting tacrolimus, or a cyclophilin and calcineurin for detecting cyclosporin, with a donor and acceptor fluorophore respectively.

The present invention also encompasses double receptor assays for tacrolimus (FK506) in which calcineurin A/B fusion proteins combined with FKBP12 are employed. The calcineurin A/B fusion proteins may be truncated sequences of the native protein with N- or C-terminal tags. Thus, in one embodiment, C-terminal his-tagged calcineurin A/B fusion proteins with molecular weights of 64 kDa (CNtAB-long) and 26 kDa (CNtAB-short), respectively, were selected for binding activity assays, based on expression and solubility screen in *E*. *coli* as well as a cell-free system. When expressed in *E*. *coli,* both CNtAB-long and CNtAB-short fusions displayed a robust expression level and the expressed fusion proteins were stable and soluble. A >90% degree of purity was achieved for both fusion proteins by a simple affinity capture and size exclusion chromatography purification procedure. Importantly, a reference time-resolved fluorescence (TRF) assay showed an EC50 value of 0.77 nM for binding of the purified CNtAB-long to FKBP12 in an FK506-dependent manner. However, the binding of the purified CNtAB-short to FKBP12-FK506 was hardly detected in that assay. A Biacore Surface Plasmon Resonance (SPR) assay confirmed the results observed by the above TRF assay.

The present invention also encompasses kits containing the assay reagents for measuring immunosuppressant drugs in samples. The kit may further include a variety of containers, e.g., vials, tubes, bottles, and the like. Preferably, the kits will also include instructions for use. In one embodiment the kit comprises first and second binding moieties wherein the first and second binding moieties specifically bind to separate binding sites on the target drug to form a sandwich complex upon contact of the reagents with the drug. The first and second binding moieties represent compounds independently selected from the group consisting of receptor proteins and antibodies. In one embodiment the kit comprises a first and second receptor protein and an antibody, wherein the first receptor protein is bound to a detection particle and the antibody specifically binds to the second receptor protein. In this embodiment the antibody specific for the second receptor can optionally be bound to a second detection particle. The individual binding moieties can be provided in separate containers or mixed together in a single container.

In one embodiment a kit is provided for detecting immunosuppressant drugs, said kit comprising a first receptor and a second receptor that specifically bind to a target immunosuppressant drug at two separate locations on the immunosuppressant drug, said first and second receptors each further comprising a tag; and a plurality of detection particles, each of said particles being bound to an agent that binds to said tag.

In a further embodiment a kit is provided for detecting an immunosuppressant drug in a sample, said kit comprising a first reagent comprising a suspension of an immunophilin bound to a first detection particle; a second reagent comprising a suspension of a receptor protein selected from the group consisting of FKBP-rapamycin associated protein, calcineurin and an antibody to calcineurin, said second receptor protein bound to a detection particle, wherein the detection particles are selected from the group consisting of microparticles and nanoshells.

In an alternative embodiment the kit comprises a first receptor and a second receptor wherein the first and second receptors are each bound to at least one detection particle, and wherein the first and second receptors specifically bind to separate binding sites on the target drug to form a sandwich complex upon contact of the receptors with the drug. In one embodiment the receptor proteins of the kit are each provided with a tag for binding the receptor to a detection particle, and the detection particles are provided in a separate container. In another embodiment the first and second receptors will be provided with the detection particle already bound to the first and second receptors. The first and second receptor proteins can be provided in separate containers or mixed together in a single container. In one embodiment the first and second receptors comprise a biotinylated tag and the detection particle is bound to streptavidin. In one embodiment each of the first and second receptors is bound to a single biotin molecule. In one embodiment the detection particle is a microparticle bound to streptavidin or to an antibody specific for biotin. In one embodiment the kit comprises two complexes, each comprising a receptor bound to a microparticle through a biotin-streptavidin linkage.

In one embodiment a kit is provided for quantitating the amount of rapamycin or everolimus in a sample. In this embodiment, the kit comprises a first receptor comprising an FK506 binding protein bound to a detection particle, and a second receptor comprising a target of rapamycin (TOR) protein bound to a second detection particle. In one embodiment the first receptor comprises FKBP12 or FKBP25 and the second receptor is selected from the group consisting of mTOR, yTOR1, yTOR2, and the FRBDs of mTOR, yTOR1, and yTOR2. In an alternative embodiment a kit is provided for quantitating the amount of tacrolimus present in a sample, wherein the kit reagents comprise a first receptor comprising an FK506 binding protein (FKBP) bound to a detection particle and a second receptor comprising calcineurin, or a binding fragment or derivative thereof, bound to a detection particle. In a further embodiment the kit further comprises calmodulin and a calcium ion, either contained in separate containers or bound to calcineurin.

In one embodiment a kit is provided for detecting active rapamycin, said kit comprising a first reagent comprising an FK506 binding protein (FKBP) linked to a first detection particle;a second reagent comprising a target of rapamycin (TOR) protein linked to a second detection particle, wherein the first and second detection particles are independently selected from the group consisting of microparticles and nanoshells. In a further embodiment a kit is provided wherein the FKBP protein is FKBP 12 or FKBP25, and wherein the FKBP-rapamycin associated protein is selected from the group consisting of mTOR, the FKBP binding domain of mTOR, yTOR1, the FKBP binding domain of yTOR1, yTOR2, and the FKBP binding domain of yTOR2.

In yet another embodiment a kit is provided for quantitating the amount of cyclosporin, wherein the reagents comprise a first receptor comprising a cyclophilin bound to a detection particle and a second receptor comprising a calcineurin bound to a detection particle. In a further embodiment the kit further comprises calmodulin and a calcium ion, either contained in separate containers or bound to calcineurin.

The detection particle of the kits is typically either a nanoshell or a microparticle, and in one embodiment the detection particle is a latex microparticle.

The following examples are set forth to illustrate specific embodiments.

### Specific embodiments

### Example 1: Synthesis of cDNA encoding yTOR1 FRBD and yTOR2 FRBD

The cDNAs encoding the 90-amino acid FKBP12 rapamycin binding domains (FRBD) of yeast TOR1 and TOR2 were chemically synthesized by Blue Heron Biotechnology (Seattle, WA) with the following optimized nucleotide acid sequence:
SEQ ID NO: 1 (yTOR1 FRBD):
SEQ ID NO: 2 (yTOR2 FRBD):

### Example 2: Construction of expression vectors

The synthetic cDNAs, along with two sets of oligonucleotide primers, were used to PCR-amplify approximately 300-bp DNA fragments, corresponding to yTOR1 FRBD and yTOR2 FRBD, and these DNA fragments were cloned into expression vector pIVEX2.7d or pIVEX2.8d (Roche Diagnostics GmbH, Germany). The resulting plasmids, confirmed by DNA sequencing, can express AviTagged target proteins that are not only able to bind rapamycin-bound FKBP12 but also to be specifically biotin-labeled at the unique lysine residue by biotin ligase BirA.
SEQ ID NO: 3 (forward primer for yTOR1 FRBD):
5' CTTTAAGAAGGAGATATACCATGGAACTTTGGTATGAAGGACTC 3'
SEQ ID NO: 4 (reverse primer for yTOR1 FRBD):
5' AAGATGTCGTTCAGGCCCCCTTGACGAGTAATTTTGCGAAA 3'
SEQ ID NO: 5 (forward primer for yTOR2 FRBD):
5' CGCTTAATTAAACATATGACCGAACAATGGTATGAAGGCCTG 3'
SEQ ID NO: 6 (reverse primer for yTOR2 FRBD):
5' TTAGTTAGTTACCGGATCCCTTACTGTTTACCGATTTTGCGAAA 3'

### Example 3: Production of biotinylated yTOR1 FRBD and biotinylated yTOR2 FRBD in E. coli

After selection using the cell-free Rapid Translation System (RTS) from Roche Diagnostics Corporation, the optimal expression constructs were transformed into BL21-A1 cells, along with BirA expression vector, and induced in the presence of 50 µM biotin, 0.2% arabinose and 0.2 mM isopropyl B-D-thiogalactopyranoside (IPTG) at 30°C for 16 hours. The expressed recombinant proteins were purified to a purity of approximately 90% through the modified avidin beads and size exclusion chromatography.

Following is the amino acid sequence for recombinant yTOR1 FRBD (106 amino acids). Lys 101 is the specific site for BirA-dependent biotin labeling. This protein binds to rapamycin at the FKBP12 binding site. The AviTag sequence is indicated by bold type, and Lys 101 **(K)** is the specific site for BirA-dependent biotin labeling.
SEQ ID NO: 7 (AviTagged yTOR1 FRBD):

Following is the amino acid sequence for recombinant yTOR2 FRBD (119 amino acids). Lys 12 is the specific site for BirA-dependent biotin labeling). This protein binds to rapamycin at the FKBP12 binding site. The AviTag sequence is indicated by bold type, and Lys 101 (**K**) is the specific site for BirA-dependent biotin labeling.
SEQ ID NO: 8: (AviTagged yTOR2 FRBD):

### Example 4: Construction of expression vectors

Two oligonucleotide primers were used to PCR-generate a DNA fragment, encoding FKBP12 with multiple glycine and histidine amino acids at the carboxyl end, were cloned into the Nco I/Sma I sites of the expression vector pIVEX2.7d. The resulting plasmid, confirmed by DNA sequencing, can express a fusion protein with an expected molecular weight of 15.0 kDa that is able to bind rapamycin, nickel NTA matrix and to be biotin-labeled at the AviTag specific Lys by biotin ligase BirA.

### Example 5: Production of biotinylated FKBP12 (MW 15 kDa) in E. coli

Transformed BL21-A1 cells harboring the above expression vector and a vector expressing BirA were induced by 0.2% arabinose and 0.4 mM isopropyl IPTG at 30°C for 16 hours. The expressed recombinant protein was purified to approximately 95% by sequential affinity purifications using Ni-NTA agarose and Mono-Avidin beads (Pierce), respectively.

Following is the amino acid sequence for this recombinant rapamycin binding protein (134 amino acids). Lys 129 is the specific site for BirA-dependent biotin labeling. This protein binds to rapamycin.
SEQ ID NO: 9: (AviTagged FKBP12):

### Example 6: Preparation of FKBP12-coated microparticles

Streptavidin microparticles (127 nm) (SA-Latex Universalreagenz ZLF, Mat. No 03140431001) were obtained from Roche Diagnostics GmbH, Germany. Final concentration was 0.3% (w/v) in 50 mM MOPS (3-(N-morpholino) propanesulfonic acid), pH 7.9.

Biotinylated FKBP12 from Example 5 (32 µL of 3.27 mg/mL) was added to 5.2 mL of 50 mM MOPS buffer (pH 7.9). This biotinylated protein solution was added to 5.2 mL of stirred suspension 0.3% (w/v) of streptavidin microparticles (127 nm) in 50 mM MOPS (pH 7.9) all at once at room temperature. The reaction was allowed to stir at room temperature for 2 hours and overnight at 4 °C, and then centrifuged at 16,000 rpm for 1 hour 45 minutes. The microparticles were resuspended in 50 mM MOPS buffer (pH 7.9) and then centrifuged at 16,000 rpm. The process of resuspension and centrifugation was repeated one more time, and the final concentration of FKBP12-coated microparticles was adjusted to 0.15% (w/v).

### Example 7: Preparation of yTOR1 FRBD- and yTOR2 FRBD-coated microparticles

Biotinylated yTOR1 FRBD from Example 3 (300 µL of 0.35 mg/mL) was added to 50 mM MOPS buffer (pH 7.9). This biotinylated protein solution was added to 5.2 mL of stirred suspension 0.3% (w/v) of streptavidin microparticles (127 nm) in 50 mM MOPS (pH 7.9) all at once at room temperature. The reaction was allowed to stir at room temperature for 2 hours and overnight at 4 °C, and then centrifuged at 16,000 rpm for 1 hour 45 minutes. The microparticles were resuspended in 50 mM MOPS buffer (pH 7.9) and then centrifuged at 16,000 rpm. The process of resuspension and centrifugation was repeated one more time, and the final concentration of yTOR1 FRBD-coated microparticles was adjusted to 0.15% (w/v).

Preparation of yTOR2 FRBD-coated microparticles using the yTOR2 FRBD from Example 3 was made as described above.

### Example 8: Rapamycin calibration curves using spiked whole blood samples

A reaction buffer reagent was prepared by making a 175 mM PIPES buffer, pH 7.4, containing 0.5% polyacrylic acid (PAA). This was a first working reagent.

Mixing a 1:1 ratio (v/v) of FKBP12-coated microparticles and yTOR1 FRBD-coated microparticles provided a second working reagent. An alternative second working reagent was prepared by mixing a 1:1 ratio (v/v) of FKBP12-coated microparticles and yTOR2 FRBD-coated microparticles.

Calibrators were made from an EDTA whole blood pool by spiking rapamycin at the levels of 0, 5, 10, 20, and 30 ng/mL using a rapamycin stock of 1 µg/mL in methanol. The pretreatment of blood samples was done using a protein precipitation reagent of a 4:1 mixture of methanol to aqueous zinc sulfate (300 mM).

Two hundred µL of whole blood was added to 200 µL of precipitation reagent and vortexed for 15-30 seconds, allowed to stand at room temperature for 5 minutes, and then spun down at 12,000 rpm for 5 minutes. The supernatant was transferred into the sample cup and loaded onto the analyzer.

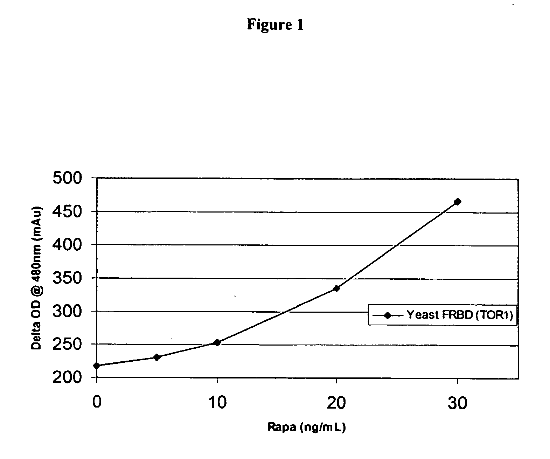

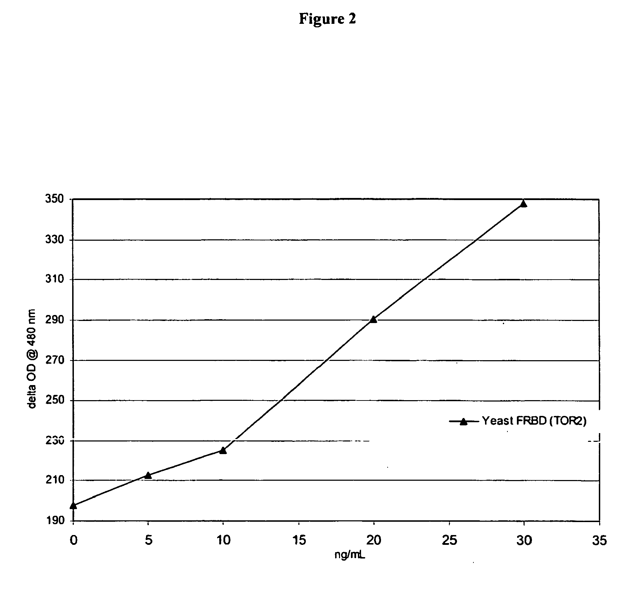

The first working reagent (reaction buffer) was placed in the R1 reagent rotor and the second working reagent (microparticle reagent) in the R2 reagent rotor. An assay was performed using a Roche/Hitachi 917 automated analyzer (Roche Diagnostics Corporation, Indianapolis) using a 35 µL sample volume, 150 µL of first working reagent and 80 µL of second working reagent (measurement at wave length 480 nm) for 10 minutes. The calibration curve for the rapamycin assay using yTOR1 FRBD is shown in Figure 1. The calibration curve for the rapamycin assay using yTOR2 FRBD is shown in Figure 2.

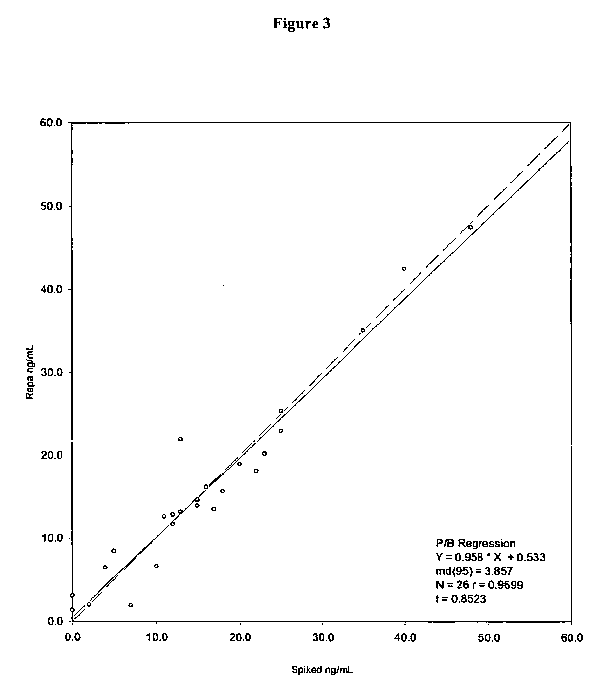

In another assay, thirteen individual whole blood samples obtained from 13 volunteers were spiked at various levels of rapamycin covering a range of 0-48 ng/mL (26 samples total were used). All 26 samples were measured according to the procedure described above using a Roche/Hitachi 917 automated analyzer. The data obtained is shown in Figure 3 and is for assay made using yTOR2 FRBD.

### Example 9: Synthesis of cDNA encoding mTOR FRBD

The cDNA encoding 90 amino acid FKBP12-rapamycin binding domain of mTOR (FRBD) was chemically synthesized by Blue Heron Biotechnology with the following optimized nucleotide acid sequence:
SEQ ID NO: 10 (mTOR FRBD):

### Example 10: Construction of expression vectors

SEQ ID NO: 11 (primer 1 for mTOR FRBD):
   5' CTTTAAGAAGGAGATATACCATGGAAATGTGGCATGAAGGACTTGAA 3'
SEQ ID NO: 12 (primer 2 for mTOR FRBD):
   5' AAGATGTCGTTCAGGCCCCCTTGTTTTGAGATACGGCGGAA 3'

The above two oligonucleotide primers and the above-described cDNA encoding mTOR FRBD were used to PCR-amplify a DNA fragment of 310 bp and was cloned into the Nco I/Sma I sites of the expression vector pIVEX2.7d. The resulting plasmid, confirmed by DNA sequencing, can express a fusion protein that is able to bind rapamycin-bound FKBP12 and can be biotin-labeled using biotin ligase BirA.

### Example 11: Production of biotinylated mTOR FRBD in E. coli

The cell-free Rapid Translation System (RTS) from Roche was used to produce the biotin-labeled, AviTagged mTOR FRBD. Purified plasmid DNA was incubated with the both RTS reaction and biotinylation reagents in an RTS 500 ProteoMaster reaction device for 18 hours. The generated recombinant protein was purified to a purity of approximately 95% through the modified avidin beads.

The amino acid sequence for this recombinant FRBD (107 amino acids) is as follows (Lys 102 is the specific site for BirA-dependent biotin labeling):
SEQ ID NO: 13 (AviTagged mTOR FRBD):

### Example 12: Preparation of FKBP12-coated microparticles

FKBP12 coated microparticles were prepared following a procedure similar to that described in Example 6. Streptavidin microparticles (SA-Latex Universalreagenz ZLF, Mat. No 03140431001) were obtained from Roche Diagnostics GmbH, Germany (127 nm). The final concentration was 0.3% (w/v) in 50 mM MOPS (3-(N-morpholino) propanesulfonic acid), pH 7.9.

Biotinylated FKBP12 (40 µL of 2.5 mg/mL) was added to 5 mL of 50 mM MOPS buffer (pH 7.9). This biotinylated protein solution was added to 5 mL of a stirred suspension of 0.3% streptavidin microparticles (127 nm) in 50 mM MOPS (pH 7.9) all at once at room temperature. The reaction was allowed to stir at room temperature for 2 hours and overnight at 4 °C, and then centrifuged at 16,000 rpm for 1 hour 45 minutes. The microparticles were resuspended in 50 mM MOPS buffer (pH 7.9) and then centrifuged at 16,000 rpm. The process of resuspension and centrifugation was repeated one more time, and the final concentration of FKBP12-coated microparticle was adjusted to 0.15% (w/v).

### Example 13: Preparation of mTOR FRBD-coated microparticles

Biotinylated m-TOR FRBD from Example 11(50 µL of 2 mg/mL) was added to 5 mL of 50 mM MOPS buffer (pH 7.9). This biotinylated protein solution was added to 5 mL of stirred suspension, 0.3%, of streptavidin microparticles (127 nm) in 50 mM MOPS (pH 7.9) all at once. The reaction was allowed to stir at room temperature for 2 hours, then centrifuged at 16,000 rpm for 1 hour 45 minutes, and the final concentration of protein-streptavidin microparticles was adjusted to 0.15% (w/v).

### Example 14: Rapamycin calibration curve using spiked whole blood samples

Mixing a 1:1 ratio (v/v) of FKBP12-coated microparticles and mTOR FRBD-coated microparticles provided a first working reagent. This reagent was used at a final concentration of 0.15% in 50 mM MOPS buffer at pH 7.9.

A second working reagent was prepared by making a 175 mM PIPES buffer, pH 7.4, containing 0.8% polyacrylic acid (PAA).

Calibrators were made by using EDTA whole blood samples spiked with 12.5, 25, and 50 ng/mL of rapamycin (from a stock of 10 µg/mL of rapamycin in methanol). The pretreatment of blood samples was done using protein precipitation with methanol and aqueous CuSO₄ (copper sulfate) solution.

200 µL of whole blood was added to 200 µL of methanol, followed immediately by 50 µL of 300 mM CuSO₄. This was vortexed for 15-30 seconds, allowed to stand at room temperature for 5 minutes, and then spun down at 12,000 rpm for 5 minutes. The supernatant was transferred to a sample cup and loaded onto a Roche/Hitachi 917 automated analyzer (Roche Diagnostics Corporation, Indianapolis).

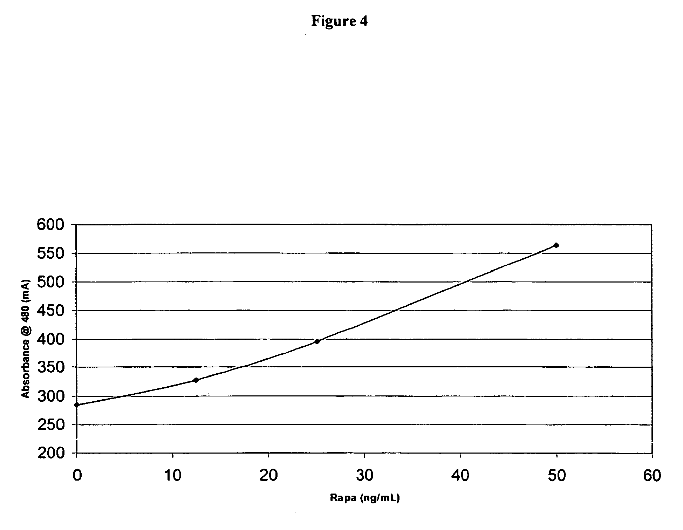

An assay was performed using a 20 µL sample volume, 80 µL of the first working reagent, 180 µL of the second working reagent, and a measurement at wave length of 480 nm. The results are shown in Figure 4.

### Example 15: Rapamycin assay using whole blood sample

A human blood sample was spiked with 25 ng/mL of rapamycin, extracted following the procedure described above, and the concentration was determined using the calibration curve shown in Figure 4 as 24.7 ng/mL and 24.6 ng/mL in duplicate runs.

### Example 16: Production of truncated recombinant calcineurin A and B fusions (CNtABs) in E. coli

### Truncated calcineurin A and B cDNAs

The cDNA encoding the truncated human calcineurin A (amino acids 12-394) and wild-type chain B (amino acids 1-170) was chemically synthesized by Blue Heron Biotechnology (Seattle, WA) with the following optimized nucleotide acid sequence (SEQ ID NO: 14):

### Construction of expression vectors

The above synthetic cDNA, along with two sets of oligonucleotide primers, were used to PCR-amplify two DNA fragments, corresponding to CNtAB-long and CNtAB-short, respectively. These DNA fragments were cloned into Roche's cell-free expression vector pIVEX2.3d in order to express two different versions of His-tagged chimeric calcineurin A/B proteins.
Forward primer for CNtAB-long (SEQ ID NO: 15):
5' CTTTAAGAAGGAGATATACCATGTCAACCACTGATCGTGTCGT 3'
Reverse primer for CNtAB-long (SEQ ID NO: 16):
5'TGATGATGAGAACCCCCCCCGACATCGACGACCATTTTTTT 3'
Forward primer for CNtAB-short (SEQ ID NO: 17):
5' CTTTAAGAAGGAGATATACCATGCCTTATTGGCTTCCCAATTTT 3'
Reverse primer for CNtAB-short (SEQ ID NO: 18):
5'TGATGATGAGAACCCCCCCCGACATCGACGACCATTTTTTT 3'

### Production of the His-tagged human calcineurin A and B fusion proteins

After pre-screening by Roche cell-free Rapid Translation System (RTS), the selected optimal expression constructs were transformed into BL21-A1 cells, and the target proteins were expressed while induced in the presence of 0.2 % arabinose and 0.4 mM IPTG at 37°C for 15 hours. The expressed recombinant protein, CNtAB-long or CNtAB-short, was purified to a purity of about 85% or about 95%, respectively, by affinity purification on Ni-NTA agarose followed by size exclusion chromatography (Superdex 200, GE).

The amino acid sequence of recombinant CNtAB-long protein (564 amino acids) is as follows (SEQ ID NO: 19):

CNtA1, corresponding to amino acids 12-394 of human calcineurin A, is in italics (at positions 2-384). CNB, corresponding to amino acids 1-170 of human calcineurin B is underlined (at positions 385-554). His tag is at positions 555-564.

The amino acid sequence of recombinant CNtAB-short (236 amino acids) is as follows (SEQ ID NO: 20):

CNtA2, corresponding to amino acids 340-394 of human calcineurin A, is in italics (at positions 2-56). CNB, corresponding to amino acids 1-170 of human calcineurin B is underlined (at positions 57-226). His tag is at positions of 227-236.

### Example 17. FK506-dependent interaction of FKBP12 with CNtAB-long

The binding activity of the truncated calcineurin A/B fusions, CNtABs, to FK506-bound FKBP12 was assessed by a reference time-resolved fluorescence assay (TRF) and other assays. In TRF assay, a black 96-well plate (Perkin Elmer) was coated overnight at 4°C with purified His-tagged CNtAB-long or CNtAB-short in 0.1 M NaHCO₃. Protein-coated plate was then blocked with 7.5% BSA in a buffer A containing 0.1 M Tris HCl, 0.15 M NaCl and 20 µM diethylenetriamine-pentaacetic acid (DTPA). Meanwhile, an FKBP12-europium (Eu) conjugate was prepared by incubation of the biotin-labeled FKBP12 from Example 5 with Eu-labeled streptavidin (Perkin-Elmer) on ice for 30 minutes. After blocking, a coated 96-well plate was washed with a buffer B containing 0.1 M Tris HCl, 0.15 M NaCl, 0.1% TWEEN (ICI Americas, Inc.) and 20 µM DTPA . The FKBP12-Eu conjugate prepared was then added to the CNtAB-long or CNtAB-short-coated plate. A series of different concentrations of FK506 were added to the plate to initiate the formation of FKBP12-FK506-CNtABs complex. After one and a half hour incubation at room temperature, the plate was washed several times with the buffer B. Finally, the enhancement solution (Perkin-Elmer) was added to the plate and incubated at room temperature with gentle shaking for 5 min. Plate was then read using a Wallac, Inc. VICTOR²V microtiter multiple plate reader. The relative fluorescence values reported are derived from the mean of triple data points.

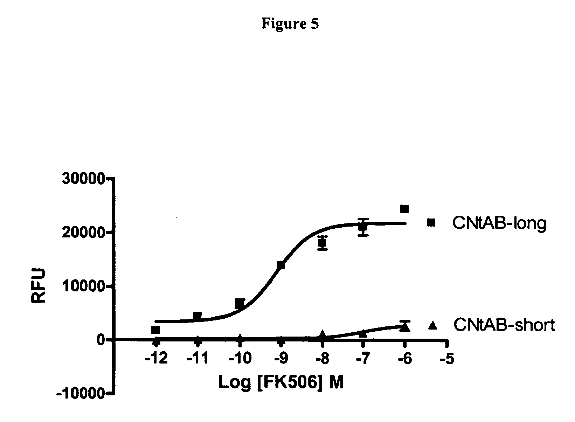

In Figure 5, an FK506 dose-dependent curve shows an EC50 value of 0.77 nM for binding of CNtAB-long to FKBP12. In contrast, the binding of CNtAB-short to FK506-bound FKBP12 is hardly detected. In addition, surface plasmon resonance BIACORE assay (Biacore AG) also is in agreement with these results observed by time-resolved fluorescence assay (data not shown). Taken together, these data indicate that the recombinant CNtAB-long is functional but that functionality of CNtAB-short is unlikely.

## Claims

1. An assay method for determining a presence or an amount of an immunosuppressant drug in a sample comprising the steps of providing a sample;mixing said sample with a first receptor and a second receptor to form a suspension, wherein said first and second receptors are bound to detection particles and each of said first and second receptors specifically bind to a separate binding site on said drug, wherein the presence of the drug results in agglutination of the detection particles; directly detecting or measuring an amount of particle agglutination in said suspension; and correlating the amount of particle agglutination with the presence or amount of the immunosuppressant drug in the sample.

2. The method of claim 1 wherein the immunosuppressant drug is selected from the group consisting of cyclosporin, tacrolimus, rapamycin, and everolimus, and said first receptor is an immunophilin or a binding fragment thereof.

3. The method of claim 1 wherein the detection particles are selected from the group consisting of microparticles and nanoshells.

4. The method of claim 1 wherein
the immunosuppressant drug is rapamycin or everolimus;
the first receptor comprises an FK506 binding protein (FKBP); and
the second receptor comprises a target of rapamycin (TOR) protein.

5. The method of claim 1 wherein
the immunosuppressant drug is tacrolimus;
the first receptor comprises an FK506 binding protein (FKBP); and
the second receptor comprises calcineurin or a binding fragment thereof.

6. The method of claim 1 wherein
the immunosuppressant drug is cyclosporin;
the first receptor comprises a cyclophilin; and
the second receptor comprises calcineurin.

7. The method of claim 6 wherein the calcineurin has both calmodulin and a calcium ion bound thereto.

8. The method of claim 3 wherein the sample is a blood sample obtained from a patient being administered at least one immunosuppressant drug.

9. A reagent for determining an immunosuppressant drug in a sample, said reagent comprising a first drug binding complex comprising a first receptor bound to a first detection particle;a second drug binding complex comprising a binding moiety selected from the group consisting of a second receptor bound to a detection particle, an immunosuppressant drug specific antibody bound to a second detection particle, and a second receptor specific antibody bound to a second detecting particle complexed with a second receptor, wherein each drug binding complex specifically binds to a separate binding site on said immunosuppressant drug.

10. The reagent of claim 9 wherein the binding moiety is a second receptor bound to a detection particle.

11. A method for determining a presence or an amount of an immunosuppressant drug in a sample comprising the steps of:
providing a sample;
mixing said sample with a receptor bound to a first detection particle and a binding moiety selected from the group consisting of an immunosuppressant drug specific antibody bound to a second detection particle, and a second receptor specific antibody complexed with a second receptor to form a suspension, wherein said receptor specifically binds to said immunosuppressant drug at a location different from where the immunosuppressant drug specific antibody binds, wherein the presence of the drug results in agglutination of the detection particles;
measuring an amount of particle agglutination in said suspension by absorbance measurement; and
correlating the amount of particle agglutination with the presence or amount of the immunosuppressant drug in the sample.

12. The method of claim 11 wherein the immunosuppressant drug is selected from the group consisting of cyclosporin, tacrolimus, rapamycin, and everolimus and the binding moiety is an antibody specific for the immunosuppressant drug, wherein the antibody is bound to a detection particle.

13. A kit for detecting immunosuppressant drugs, said kit comprising a first receptor and a second receptor that specifically bind to a target immunosuppressant drug at two separate locations on the immunosuppressant drug, said first and second receptors each further comprising a tag; and a plurality of detection particles, each of said particles being bound to an agent that binds to said tag.

14. A kit for detecting an immunosuppressant drug in a sample, said kit comprising a first reagent comprising a suspension of an immunophilin bound to a first detection particle; a second reagent comprising a suspension of a receptor protein selected from the group consisting of FKBP-rapamycin associated protein, calcineurin and an antibody to calcineurin, said second receptor protein bound to a detection particle, wherein the detection particles are selected from the group consisting of microparticles and nanoshells.

15. A kit for detecting active rapamycin, said kit comprising
a first reagent comprising an FK506 binding protein (FKBP) linked to a first detection particle;
a second reagent comprising a target of rapamycin (TOR) protein linked to a second detection particle, wherein the first and second detection particles are independently selected from the group consisting of microparticles and nanoshells.

## Patentansprüche

1. Testverfahren zur Bestimmung eines Vorliegens oder einer Menge eines Immunsuppressivums in einer Probe, mit den folgenden Schritten: Bereitstellen einer Probe; Mischen der Probe mit einem ersten Rezeptor und einem zweiten Rezeptor unter Bildung einer Suspension, wobei der erste und der zweite Rezeptor an Nachweispartikel gebunden sind und jeweils spezifisch an eine separate Bindungsstelle auf dem Immunsuppressivum binden, wobei das Vorliegen des Immunsuppressivums zur Agglutination der Nachweispartikel führt; direktes Nachweisen oder Messen einer Menge an Partikelagglutination in der Suspension; und Korrelieren der Menge an Partikelagglutination mit dem Vorliegen oder der Menge des Immunsuppressivums in der Probe.

2. Verfahren nach Anspruch 1, wobei das Immunsuppressivum ausgewählt ist aus der Gruppe bestehend aus Cyclosporin, Tacrolimus, Rapamycin und Everolimus und es sich bei dem ersten Rezeptor um ein Immunophilin oder ein Bindungsfragment davon handelt.

3. Verfahren nach Anspruch 1, wobei die Nachweispartikel ausgewählt sind aus der Gruppe bestehend aus Mikropartikeln und Nanohüllen.

4. Verfahren nach Anspruch 1, wobei
es sich bei dem Immunsuppressivum um Rapamycin oder Everolimus handelt;
der erste Rezeptor ein FK506-Bindungsprotein (FKBP) umfasst; und
der zweite Rezeptor ein TOR(Target of Rapamycin)-Protein umfasst.

5. Verfahren nach Anspruch 1, wobei
es sich bei dem Immunsuppressivum um Tacrolimus handelt;
der erste Rezeptor ein FK506-Bindungsprotein (FKBP) umfasst; und
der zweite Rezeptor Calcineurin oder ein Bindungsfragment davon umfasst.

6. Verfahren nach Anspruch 1, wobei
es sich bei dem Immunsuppressivum um Cyclosporin handelt;
der erste Rezeptor ein Cyclophilin umfasst; und
der zweite Rezeptor Calcineurin umfasst.

7. Verfahren nach Anspruch 6, wobei an dem Calcineurin sowohl Calmodulin als auch ein Calciumion gebunden ist.

8. Verfahren nach Anspruch 3, wobei es sich bei der Probe um eine einem Patienten, dem wenigstens ein Immunsuppressivum verabreicht wurde, entnommene Blutprobe handelt.

9. Reagens zur Bestimmung eines Immunsuppressivums in einer Probe, wobei das Reagens einen ersten Arzneistoffbindungskomplex, der einen an ein erstes Nachweispartikel gebundenen ersten Rezeptor umfasst, einen zweiten Arzneistoffbindungskomplex, der eine aus der Gruppe bestehend aus einem an ein Nachweispartikel gebundenen zweiten Rezeptor, einem an ein zweites Nachweispartikel gebundenen, für ein Immunsuppressivum spezifischen Antikörper und einem an ein mit einem zweiten Rezeptor komplexiertes zweites Nachweispartikel gebundenen, für den zweiten Rezeptor spezifischen Antikörper ausgewählte Bindungsgruppierung umfasst, wobei die Arzneistoffbindungskomplexe jeweils spezifisch an eine separate Bindungsstelle auf dem Immunsuppressivum binden.

10. Reagens nach Anspruch 9, wobei es sich bei der Bindungsgruppierung um einen an ein Nachweispartikel gebundenen zweiten Rezeptor handelt.

11. Verfahren zur Bestimmung eines Vorliegens oder einer Menge eines Immunsuppressivums in einer Probe, mit den folgenden Schritten:
Bereitstellen einer Probe;
Mischen der Probe mit einem an ein erstes Nachweispartikel gebundenen Rezeptor und einer aus der Gruppe bestehend aus einem an ein zweites Nachweispartikel gebundenen, für ein Immunsuppressivum spezifischen Antikörper und einem mit einem zweiten Rezeptor komplexierten, für den zweiten Rezeptor spezifischen Antikörper ausgewählten Bindungsgruppierung unter Bildung einer Suspension, wobei der Rezeptor spezifisch an das Immunsuppressivum an einem anderen Ort als der für ein Immunsuppressivum spezifische Antikörper bindet, wobei das Vorliegen des Immunsuppressivums zur Agglutination der Nachweispartikel führt;
Messen einer Menge an Partikelagglutination in der Suspension mittels Absorptionsmessung; und
Korrelieren der Menge an Partikelagglutination mit dem Vorliegen oder der Menge des Immunsuppressivums in der Probe.

12. Verfahren nach Anspruch 11, wobei das Immunsuppressivum ausgewählt ist aus der Gruppe bestehend aus Cyclosporin, Tacrolimus, Rapamycin und Everolimus und es sich bei der Bindungsgruppierung um einen für das Immunsuppressivum spezifischen Antikörper handelt, wobei der Antikörper an ein Nachweispartikel gebunden ist.

13. Kit zum Nachweisen von Immunsuppressiva, wobei das Kit
einen ersten Rezeptor und einen zweiten Rezeptor, die spezifisch an ein Ziel-Immunsuppressivum an zwei separaten Orten auf dem Immunsuppressivum binden, wobei der erste und der zweite Rezeptor ferner jeweils ein Tag umfassen, und mehrere Nachweispartikel, die jeweils an ein Agens, das an das Tag bindet, gebunden sind, umfasst.

14. Kit zum Nachweisen eines Immunsuppressivums in einer Probe, wobei das Kit
ein erstes Reagens, das eine Suspension eines an ein erstes Nachweispartikel gebundenen Immunophilins umfasst, ein zweites Reagens, das eine Suspension eines Rezeptorproteins, ausgewählt aus der Gruppe bestehend aus FKBP-Rapamycin-assoziiertem Protein, Calcineurin und einem Antikörper gegen Calcineurin, umfasst, wobei das zweite Rezeptorprotein an ein Nachweispartikel gebunden ist, wobei die Nachweispartikel ausgewählt sind aus der Gruppe bestehend aus Mikropartikeln und Nanohüllen, umfasst.

15. Kit zum Nachweisen von aktivem Rapamycin, wobei das Kit
ein erstes Reagens, das ein FK506-Bindungsprotein (FKBP) in Verknüpfung mit einem ersten Nachweispartikel umfasst;
ein zweites Reagens, das ein TOR(Target of Rapamycin)-Protein in Verknüpfung mit einem zweiten Nachweispartikel umfasst, wobei das erste und das zweite Nachweispartikel jeweils unabhängig ausgewählt sind aus der Gruppe bestehend aus Mikropartikeln und Nanohüllen, umfasst.

## Revendications

1. Procédé de dosage pour déterminer une présence ou une quantité d'un médicament immunosuppresseur dans un échantillon, comprenant les étapes dans lesquelles : on procure un échantillon ; on mélange ledit échantillon avec un premier récepteur et avec un deuxième récepteur pour obtenir une suspension, lesdits premier et deuxième récepteurs étant liés à des particules de détection et chacun desdits premier et deuxième récepteurs se liant de manière spécifique à un site de liaison séparé sur ledit médicament, la présence du médicament donnant lieu à une agglutination des particules de détection ; on détecte ou on mesure directement une quantité d'agglutination de particules dans ladite suspension ; et on établit une corrélation entre la quantité d'agglutination de particules et la présence ou la quantité du médicament immunosuppresseur dans l'échantillon.

2. Procédé selon la revendication 1, dans lequel le médicament immunosuppresseur est choisi parmi le groupe constitué par la cyclosporine, le tacrolimus, la rapamycine et l'évérolimus, et ledit premier récepteur est une immunophiline ou un fragment de liaison de cette dernière.

3. Procédé selon la revendication 1, dans lequel les particules de détection sont choisies parmi le groupe constitué par des microparticules et des nanobilles.

4. Procédé selon la revendication 1, dans lequel
le médicament immunosuppresseur est la rapamycine ou l'évérolimus ; le premier récepteur comprend une protéine de liaison à FK506 (FKBP) ; et
le deuxième récepteur comprend une cible de la protéine rapamycine (TOR).

5. Procédé selon la revendication 1, dans lequel
le médicament immunosuppresseur est le tacrolimus ;
le premier récepteur comprend une protéine de liaison à FK506 (FKBP) ; et
le deuxième récepteur comprend de la calcineurine ou un fragment de liaison de cette dernière.

6. Procédé selon la revendication 1, dans lequel
le médicament immunosuppresseur est la cyclosporine ;
le premier récepteur comprend une cyclophiline ; et
le deuxième récepteur comprend de la calcineurine.

7. Procédé selon la revendication 6, dans lequel, à la calcineurine, sont liés à la fois de la calmoduline et un ion de calcium.

8. Procédé selon la revendication 3, dans lequel l'échantillon est un échantillon de sang que l'on obtient à partir d'un patient auquel on administre au moins un médicament immunosuppresseur.

9. Réactif pour déterminer un médicament immunosuppresseur dans un échantillon, ledit réactif comprenant un premier complexe de liaison à un médicament comprenant un premier récepteur lié à une première particule de détection, un deuxième complexe de liaison à un médicament comprenant une fraction de liaison choisie parmi le groupe constitué par un deuxième récepteur lié à une particule de détection, un anticorps spécifique à un médicament immunosuppresseur lié à une deuxième particule de détection, et un anticorps, spécifique à un deuxième récepteur, lié à une deuxième particule de détection, complexé à un deuxième récepteur, chaque complexe de liaison à un médicament se liant de manière spécifique à un site de liaison séparé sur ledit médicament immunosuppresseur.

10. Réactif selon la revendication 9, dans lequel la fraction de liaison est un deuxième récepteur lié à une particule de détection.

11. Procédé pour la détermination d'une présence ou d'une quantité d'un médicament immunosuppresseur dans un échantillon, comprenant les étapes dans lesquelles :
on procure un échantillon ;
on mélange ledit échantillon avec un récepteur lié à une première particule de détection et une fraction de liaison choisie parmi le groupe constitué par un anticorps spécifique à un médicament immunosuppresseur lié à une deuxième particule de détection et un anticorps spécifique à un deuxième récepteur, complexé avec un deuxième récepteur pour obtenir une suspension, ledit récepteur se liant de manière spécifique audit médicament immunosuppresseur à un endroit différent de l'endroit auquel se lie l'anticorps spécifique au médicament immunosuppresseur, la présence du médicament donnant lieu à une agglutination des particules de détection ;
on mesure une quantité d'agglutination de particules dans ladite suspension via une mesure de l'absorbance ; et
on établit une corrélation entre la quantité d'agglutination des particules et la présence ou la quantité du médicament immunosuppresseur dans l'échantillon.

12. Procédé selon la revendication 11, dans lequel le médicament immunosuppresseur est choisi parmi le groupe constitué par la cyclosporine, le tacrolimus, la rapamycine et l'évérolimus, et la fraction de liaison est un anticorps spécifique pour le médicament immunosuppresseur, l'anticorps étant lié à une particule de détection.

13. Nécessaire pour la détection de médicaments immunosuppresseurs, ledit nécessaire comprenant un premier récepteur et un deuxième récepteur qui se lient de manière spécifique à un médicament immunosuppresseur cible à deux endroits séparés sur le médicament immunosuppresseur, lesdits premier et deuxième récepteurs comprenant en outre chacun une étiquette ; et plusieurs particules de détection, chacune desdites particules étant liée à un agent qui se lie à ladite étiquette.

14. Nécessaire pour la détection d'un médicament immunosuppresseur dans un échantillon, ledit nécessaire comprenant un premier réactif comprenant une suspension d'une immunophiline liée à une première particule de détection ;
un deuxième réactif comprenant une suspension d'une protéine récepteur choisie parmi le groupe constitué par la protéine associée à la FKBP-rapamycine, la calcineurine et un anticorps dirigé contre la calcineurine, ladite deuxième protéine récepteur étant liée à une particule de détection, les particules de détection étant choisies parmi le groupe constitué par des microparticules et des nanobilles.

15. Nécessaire pour détecter une rapamycine active, ledit nécessaire comprenant
un premier réactif comprenant une protéine de liaison à FK506 (FKBP) liée à une première particule de détection ;
un deuxième réactif comprenant une cible d'une protéine de rapamycine (TOR) liée à une deuxième particule de détection, lesdites première et deuxième particules de détection étant choisies de manière indépendante parmi le groupe constitué par des microparticules et par des nanobilles.


### SEQUENCE LISTING

<110> SIGLER, GERALD F.
   TSAI, JANE S. C.
   GHOSHAL, MITALI
   RASHID, SHAKER
   WU, YIFEI
   DORN, ALLAN
<120> HOMOGENEOUS DOUBLE RECEPTOR AGGLUTINATION ASSAY FOR IMMUNOSUPPRESSANT DRUGS
<130> 23514 WO
<140>
   <141>
<150> 60/807,245
   <151> 2006-07-13
<160> 20
<170> PatentIn Ver. 3.3
<210> 1
   <211> 270
   <212> DNA
   <213> Artificial Sequence
<220>
   <223> Description of Artificial Sequence: synthetic construct
<400> 1
   gaactttggt atgaaggact cgaagatgca tctcgccaat tttttgttga acacaatatc 60
   gaaaaaatgt tttctacact tgaaccctta cacaaacacc ttggaaatga accacaaacc 120
   ctctctgaag tctcctttca aaaatccttt ggccgtgacc ttaacgacgc atatgaatgg 180
   cttaacaact acaaaaaatc taaagatatt aataacctga atcaagcatg ggacatctat 240
   tacaacgtat ttcgcaaaat tactcgtcaa                                  270
<210> 2
   <211> 270
   <212> DNA
   <213> Artificial Sequence
<220>
   <223> Description of Artificial Sequence: Synthetic construct
<400> 2
   gaacaatggt atgaaggcct ggatgatgcc tctcgtcaat tctttggtga acacaataca 60
   gaaaaaatgt ttgcagcgct cgaaccctta tatgaaatgt taaaacgtgg tccagaaact 120
   ttacgggaaa tttcttttca aaattctttt ggtcgtgatc tcaatgatgc ctatgaatgg 180
   ttaatgaatt ataaaaaaag caaagatgtt agcaacctta atcaagcctg ggatatttac 240
   tataacgtat ttcgcaaaat cggtaaacag                                  270
<210> 3
   <211> 44
   <212> DNA
   <213> Artificial Sequence
<220>
   <223> Description of Artificial Sequence: Synthetic primer
<400> 3
   ctttaagaag gagatatacc atggaacttt ggtatgaagg actc 44
<210> 4
   <211> 41
   <212> DNA
   <213> Artificial Sequence
<220>
   <223> Description of Artificial Sequence: Synthetic primer
<400> 4
   aagatgtcgt tcaggccccc ttgacgagta attttgcgaa a 41
<210> 5
   <211> 42
   <212> DNA
   <213> Artificial Sequence
<220>
   <223> Description of Artificial Sequence: synthetic primer
<400> 5
   cgcttaatta aacatatgac cgaacaatgg tatgaaggcc tg 42
<210> 6
   <211> 44
   <212> DNA
   <213> Artificial Sequence
<220>
   <223> Description of Artificial Sequence: Synthetic primer
<400> 6
   ttagttagtt accggatccc ttactgttta ccgattttgc gaaa 44
<210> 7
   <211> 106
   <212> PRT
   <213> Artificial Sequence
<220>
   <223> Description of Artificial Sequence: Synthetic construct
<400> 7
   Glu Leu Trp Tyr Glu Gly Leu Glu Asp Ala Ser Arg Gln Phe Phe Val
   1               5                   10                  15
   Glu His Asn Ile Glu Lys Met Phe Ser Thr Leu Glu Pro Leu His Lys
               20                  25                  30
   His Leu Gly Asn Glu Pro Gln Thr Leu Ser Glu Val Ser Phe Gln Lys
           35                  40                  45
   Ser Phe Gly Arg Asp Leu Asn Asp Ala Tyr Glu Trp Leu Asn Asn Tyr
       50                  55                  60
   Lys Lys Ser Lys Asp Ile Asn Asn Leu Asn Gln Ala Trp Asp Ile Tyr
   65                  70                  75                  80
   Tyr Asn Val Phe Arg Lys Ile Thr Arg Gln Gly Gly Leu Asn Asp Ile
                   85                  90                  95
   Phe Glu Ala Gln Lys Ile Glu Trp His Glu
               100                 105
<210> 8
   <211> 119
   <212> PRT
   <213> Artificial Sequence
<220>
   <223> Description of Artificial Sequence: Synthetic construct
<400> 8
   Met Ser Gly Leu Asn Asp Ile Phe Glu Ala Gln Lys Ile Glu Trp His
   1               5                   10                  15
   Glu Ile Glu Gly Arg Gly Arg Leu Ile Lys His Met Thr Glu Gln Trp
               20                  25                  30
   Tyr Glu Gly Leu Asp Asp Ala Ser Arg Gln Phe Phe Gly Glu His Asn
           35                  40                  45
   Thr Glu Lys Met Phe Ala Ala Leu Glu Pro Leu Tyr Glu Met Leu Lys
       50                  55                  60
   Arg Gly Pro Glu Thr Leu Arg Glu Ile Ser Phe Gln Asn Ser Phe Gly
   65                  70                  75                  80
   Arg Asp Leu Asn Asp Ala Tyr Glu Trp Leu Met Asn Tyr Lys Lys Ser
                   85                  90                  95
   Lys Asp Val Ser Asn Leu Asn Gln Ala Trp Asp Ile Tyr Tyr Asn Val
               100                 105                 110
   Phe Arg Lys Ile Gly Lys Gln
           115
<210> 9
   <211> 134
   <212> PRT
   <213> Artificial Sequence
<220>
   <223> Description of Artificial sequence: Synthetic construct
<400> 9
   Met Gly Val Gln Val Glu Thr Ile Ser Pro Gly Asp Gly Arg Thr Phe
   1               5                   10                  15
   Pro Lys Arg Gly Gln Thr Cys Val Val His Tyr Thr Gly Met Leu Glu
               20                  25                  30
   Asp Gly Lys Lys Phe Asp Ser Ser Arg Asp Arg Asn Lys Pro Phe Lys
           35                  40                  45
   Phe Met Leu Gly Lys Gln Glu Val Ile Arg Gly Trp Glu Glu Gly Val
       50                  55                  60
   Ala Gln Met Ser Val Gly Gln Arg Ala Lys Leu Thr Ile Ser Pro Asp
   65                  70                  75                  80
   Tyr Ala Tyr Gly Ala Thr Gly His Pro Gly Ile Ile Pro Pro His Ala
                   85                  90                  95
   Thr Leu Val Phe Asp Val Glu Leu Leu Lys Leu Glu Gly Gly Gly Ser
               100                 105                 110
   His His His His His His Gly Gly Leu Asn Asp Ile Phe Glu Ala Gln
           115                 120                 125
   Lys Ile Glu Trp His Glu
       130
<210> 10
   <211> 270
   <212> DNA
   <213> Artificial Sequence
<220>
   <223> Description of Artificial Sequence: Synthetic construct
<400> 10
   gaaatgtggc atgaaggact tgaagaagct agccgtctct attttggtga acgcaacgta 60
   aaaggaatgt ttgaagtact tgaaccttta cacgcaatga tggaacgtgg accccaaacc 120
   ttaaaagaaa cctcctttaa tcaagcatat ggtcgtgact taatggaagc tcaagaatgg 180
   tgtcgtaaat atatgaaatc tggtaatgta aaagatttaa ctcaagcctg ggatttatac 240
   tatcacgttt tccgccgtat ctcaaaacaa                                  270
<210> 11
   <211> 47
   <212> DNA
   <213> Artificial Sequence
<220>
   <223> Description of Artificial sequence: Synthetic primer
<400> 11
   ctttaagaag gagatatacc atggaaatgt ggcatgaagg acttgaa 47
<210> 12
   <211> 41
   <212> DNA
   <213> Artificial Sequence
<220>
   <223> Description of Artificial Sequence: Synthetic primer
<400> 12
   aagatgtcgt tcaggccccc ttgttttgag atacggcgga a 41
<210> 13
   <211> 107
   <212> PRT
   <213> Artificial Sequence
<220>
   <223> Description of Artificial Sequence: Synthetic construct
<400> 13
   Met Glu Met Trp His Glu Gly Leu Glu Glu Ala Ser Arg Leu Tyr Phe
   1               5                   10                  15
   Gly Glu Arg Asn Val Lys Gly Met Phe Glu Val Leu Glu Pro Leu His
                   20                  25                  30
   Ala Met Met Glu Arg Gly Pro Gln Thr Leu Lys Glu Thr Ser Phe Asn
           35                  40                  45
   Gln Ala Tyr Gly Arg Asp Leu Met Glu Ala Gln Glu Trp Cys Arg Lys
       50                  55                  60
   Tyr Met Lys Ser Gly Asn Val Lys Asp Leu Thr Gln Ala Trp Asp Leu
   65                  70                  75                  80
   Tyr Tyr His Val Phe Arg Arg Ile Ser Lys Gln Gly Gly Leu Asn Asp
                   85                  90                  95
   Ile Phe Glu Ala Gln Lys Ile Glu Trp His Glu
               100                 105
<210> 14
   <211> 1659
   <212> DNA
   <213> Artificial Sequence
<220>
   <223> Description of Artificial Sequence: Synthetic construct
<400> 14
   tcaaccactg atcgtgtcgt taaagctgtc ccgtttccac cgagccaccg cttaactgca 60
   aaagaagttt ttgataacga cggaaaaccg cgtgttgata tccttaaagc acatctgatg 120
   aaggaaggcc gtttagaaga atcagtagcg ctgcgtatca ttaccgaagg agcgtcaatc 180
   ttacgtcaag aaaaaaatct gctcgacatc gatgcaccgg ttaccgtatg tggtgatatt 240
   catggtcagt ttttcgactt aatgaaatta tttgaagtgg gtggttctcc ggctaacact 300
   cgttatctct ttctgggtga ttacgtcgat cgtggctact tttctattga gtgtgttctg 360
   tacctgtggg cattaaaaat tctttatcca aaaactcttt tcttactgcg tggcaatcat 420
   gaatgtcgtc atctgaccga atacttcact tttaaacaag aatgtaaaat caaatatagc 480
   gaacgtgtgt atgatgcttg tatggatgcc tttgattgct tacccttagc cgccttaatg 540
   aaccaacaat tcctgtgtgt acatggtggc cttagccccg aaattaacac cttagatgat 600
   atccgtaaat tagatcgttt taaagaacca cccgcttatg gcccgatgtg tgatatcttg 660
   tggtcggatc ctctggaaga ttttggtaac gaaaaaactc aggaacattt tactcataac 720
   accgttcgtg gctgtagtta tttctattct tacccagccg tgtgcgaatt ccttcaacac 780
   aacaacctgc tgagtattct ccgcgcccat gaagcccaag atgccggtta tcgtatgtat 840
   cgtaaatcac agaccaccgg gttcccatct ctcattacga ttttttctgc cccgaactac 900
   cttgacgttt acaataataa agccgctgtt ctgaaatatg aaaacaacgt catgaatatt 960
   cgccaattca attgcagccc tcacccttat tggcttccca attttatgga cgtgtttacc 1020
   tggtcactgc catttgtcgg tgagaaagtt actgaaatgc ttgttaatgt tctgaacatc 1080
   tgtagcgatg atgaattagg gtccgaagaa gacgggtttg acggtgccac agctgccgcg 1140
   cgcaaagaaa tgggcaacga agcatcctat ccacttgaaa tgtgttctca ttttgatgcc 1200
   gatgaaatta aacgcttagg taaacggttt aaaaaacttg atcttgataa ctctggctcc 1260
   ctttctgtgg aagaatttat gtccttaccg gagctgcagc agaaccctct ggtccaacgc 1320
   gtaattgata tctttgacac cgatggaaat ggcgaagtcg attttaaaga atttattgaa 1380
   ggtgtttctc aatttagcgt aaagggtgac aaagaacaga aactgcgttt cgcattccgc 1440
   atctatgaca tggataaaga tggttatatc tcaaacgggg aattgttcca ggttttaaaa 1500
   atgatggtag gcaacaattt aaaagatacc caattacaac aaattgtaga taaaacgatt 1560
   attaacgcag ataaggatgg tgatggtcgc atttctttcg aagagttctg cgccgttgtg 1620
   ggaggtcttg atattcataa aaaaatggtc gtcgatgtc                        1659
<210> 15
   <211> 43
   <212> DNA
   <213> Artificial Sequence
<220>
   <223> Description of Artificial Sequence: Synthetic primer
<400> 15
   ctttaagaag gagatatacc atgtcaacca ctgatcgtgt cgt 43
<210> 16
   <211> 41
   <212> DNA
   <213> Artificial Sequence
<220>
   <223> Description of Artificial sequence: Synthetic primer
<400> 16
   tgatgatgag aacccccccc gacatcgacg accatttttt t 41
<210> 17
   <211> 44
   <212> DNA
   <213> Artificial Sequence
<220>
   <223> Description of Artificial Sequence: Synthetic primer <400> 17

   ctttaagaag gagatatacc atgccttatt ggcttcccaa tttt 44
<210> 18
   <211> 41
   <212> DNA
   <213> Artificial sequence
<220>
   <223> Description of Artificial Sequence: Synthetic primer
<400> 18
   tgatgatgag aacccccccc gacatcgacg accatttttt t 41
<210> 19
   <211> 564
   <212> PRT
   <213> Artificial Sequence
<220>
   <223> Description of Artificial Sequence: Synthetic construct
<400> 19
   Met Ser Thr Thr Asp Arg Val Val Lys Ala Val Pro Phe Pro Pro Ser
   1               5                   10                  15
   His Arg Leu Thr Ala Lys Glu Val Phe Asp Asn Asp Gly Lys Pro Arg
               20                  25                  30
   Val Asp Ile Leu Lys Ala His Leu Met Lys Glu Gly Arg Leu Glu Glu
           35                  40                  45
   Ser Val Ala Leu Arg Ile Ile Thr Glu Gly Ala Ser Ile Leu Arg Gln
       50                  55                  60
   Glu Lys Asn Leu Leu Asp Ile Asp Ala Pro Val Thr Val Cys Gly Asp
   65                  70                  75                  80
   Ile His Gly Gln Phe Phe Asp Leu Met Lys Leu Phe Glu Val Gly Gly
                       85                  90                  95
   Ser Pro Ala Asn Thr Arg Tyr Leu Phe Leu Gly Asp Tyr Val Asp Arg
               100                 105                 110
   Gly Tyr Phe Ser Ile Glu Cys Val Leu Tyr Leu Trp Ala Leu Lys Ile
           115                 120                 125
   Leu Tyr Pro Lys Thr Leu Phe Leu Leu Arg Gly Asn His Glu Cys Arg
       130                 135                 140
   His Leu Thr Glu Tyr Phe Thr Phe Lys Gln Glu Cys Lys Ile Lys Tyr
   145                 150                 155                 160
   Ser Glu Arg Val Tyr Asp Ala Cys Met Asp Ala Phe Asp Cys Leu Pro
                   165                 170                 175
   Leu Ala Ala Leu Met Asn Gln Gln Phe Leu Cys Val His Gly Gly Leu
               180                 185                 190
   Ser Pro Glu Ile Asn Thr Leu Asp Asp Ile Arg Lys Leu Asp Arg Phe
           195                 200                 205
   Lys Glu Pro Pro Ala Tyr Gly Pro Met Cys Asp Ile Leu Trp Ser Asp
       210                 215                 220
   Pro Leu Glu Asp Phe Gly Asn Glu Lys Thr Gln Glu His Phe Thr His
   225                 230                 235                 240
   Asn Thr Val Arg Gly Cys Ser Tyr Phe Tyr Ser Tyr Pro Ala Val Cys
                   245                 250                 255
   Glu Phe Leu Gln His Asn Asn Leu Leu Ser Ile Leu Arg Ala His Glu
               260                 265                 270
   Ala Gln Asp Ala Gly Tyr Arg Met Tyr Arg Lys Ser Gln Thr Thr Gly
           275                 280                 285
   Phe Pro Ser Leu Ile Thr Ile Phe Ser Ala Pro Asn Tyr Leu Asp Val
       290                 295                 300
   Tyr Asn Asn Lys Ala Ala Val Leu Lys Tyr Glu Asn Asn Val Met Asn
   305                 310                 315                 320
   Ile Arg Gln Phe Asn Cys Ser Pro His Pro Tyr Trp Leu Pro Asn Phe
                   325                 330                 335
   Met Asp Val Phe Thr Trp Ser Leu Pro Phe Val Gly Glu Lys Val Thr
               340                 345                 350
   Glu Met Leu Val Asn Val Leu Asn Ile Cys Ser Asp Asp Glu Leu Gly
           355                 360                 365
   Ser Glu Glu Asp Gly Phe Asp Gly Ala Thr Ala Ala Ala Arg Lys Glu
       370                 375                 380
   Met Gly Asn Glu Ala Ser Tyr Pro Leu Glu Met Cys Ser His Phe Asp
   385                 390                 395                 400
   Ala Asp Glu Ile Lys Arg Leu Gly Lys Arg Phe Lys Lys Leu Asp Leu
                   405                 410                 415
   Asp Asn Ser Gly Ser Leu Ser Val Glu Glu Phe Met Ser Leu Pro Glu
               420                 425                 430
   Leu Gln Gln Asn Pro Leu Val Gln Arg Val Ile Asp Ile Phe Asp Thr
           435                 440                 445
   Asp Gly Asn Gly Glu Val Asp Phe Lys Glu Phe Ile Glu Gly Val Ser
   450 455 460
   Gln Phe Ser Val Lys Gly Asp Lys Glu Gln Lys Leu Arg Phe Ala Phe
   465 470 475 480
   Arg Ile Tyr Asp Met Asp Lys Asp Gly Tyr Ile Ser Asn Gly Glu Leu
   485 490 495
   Phe Gln Val Leu Lys Met Met Val Gly Asn Asn Leu Lys Asp Thr Gln
   500 505 510
   Leu Gln Gln Ile Val Asp Lys Thr Ile Ile Asn Ala Asp Lys Asp Gly
   515 520 525
   Asp Gly Arg Ile Ser Phe Glu Glu Phe Cys Ala Val Val Gly Gly Leu
   530 535 540
   Asp Ile His Lys Lys Met Val Val Asp Val Gly Gly Gly Ser His His
   545 550 555 560
   His His His His
<210> 20
   <211> 236
   <212> PRT
   <213> Artificial Sequence
<220>
   <223> Description of Artificial Sequence: Synthetic construct
<400> 20
   Met Pro Tyr Trp Leu Pro Asn Phe Met Asp Val Phe Thr Trp Ser Leu
   1               5                   10                  15
   Pro Phe Val Gly Glu Lys Val Thr Glu Met Leu Val Asn Val Leu Asn
               20                  25                  30
   Ile Cys Ser Asp Asp Glu Leu Gly Ser Glu Glu Asp Gly Phe Asp Gly
           35                  40                  45
   Ala Thr Ala Ala Ala Arg Lys Glu Met Gly Asn Glu Ala Ser Tyr Pro
       50                  55                  60
   Leu Glu Met Cys Ser His Phe Asp Ala Asp Glu Ile Lys Arg Leu Gly
   65                  70                  75                  80
   Lys Arg Phe Lys Lys Leu Asp Leu Asp Asn Ser Gly Ser Leu Ser Val
                   85                  90                  95
   Glu Glu Phe Met Ser Leu Pro Glu Leu Gln Gln Asn Pro Leu Val Gln
               100                 105                 110
   Arg Val Ile Asp Ile Phe Asp Thr Asp Gly Asn Gly Glu Val Asp Phe
           115                 120                 125
   Lys Glu Phe Ile Glu Gly Val Ser Gln Phe Ser Val Lys Gly Asp Lys
       130                 135                 140
   Glu Gln Lys Leu Arg Phe Ala Phe Arg Ile Tyr Asp Met Asp Lys Asp
   145                 150                 155                 160
   Gly Tyr Ile Ser Asn Gly Glu Leu Phe Gln Val Leu Lys Met Met Val
                   165                 170                 175
   Gly Asn Asn Leu Lys Asp Thr Gln Leu Gln Gln Ile Val Asp Lys Thr
               180                 185                 190
   Ile Ile Asn Ala Asp Lys Asp Gly Asp Gly Arg Ile Ser Phe Glu Glu
           195                 200                 205
   Phe Cys Ala Val Val Gly Gly Leu Asp Ile His Lys Lys Met Val Val
       210                 215                 220
   Asp Val Gly Gly Gly Ser His His His His His His
   225                 230                 235